# EUROPEAN PATENT SPECIFICATION

(11) **EP 4 487 341 B1**
(45) Date of publication and mention of the grant of the patent: **24.06.2026**
(21) Application number: 23713026.5
(22) Date of filing: 03.03.2023
(51) Int. Cl.: G16H 20/30, A63F 13/00, A63F 13/79, G09B 1/00, G16H 20/70, G16H 40/63, G16H 50/30, G09B 5/00, G09B 19/00, G16H 10/20, A63F 13/212, A63F 13/47, A63F 13/798

(54) **TREATMENT CONTENT DELIVERY AND PROGRESS TRACKING SYSTEM**
SYSTEM ZUR BEREITSTELLUNG VON BEHANDLUNGSINHALTEN UND ZUR VERFOLGUNG DES FORTSCHRITTS
SYSTÈME DE DISTRIBUTION DE CONTENU DE TRAITEMENT ET DE SUIVI DE PROGRESSION

(30) Priority: 04.03.2022 US 202263268905 P; 05.04.2022 US 202263362497 P; 26.04.2022 US 202263363639 P; 16.06.2022 US 202263366521 P
(43) Date of publication of application: 08.01.2025
(73) Proprietor: Deepwell DTX, Las Vegas, Nevada 89118 (US)
(72) Inventor: DOUGLAS, Ryan, Seattle, Washington 98107 (US)
(74) Representative: Schweiger, Martin
(86) International application number: PCT/US2023/063720
(87) International publication number: WO 2023/168435

(56) References cited:
- WO-A1-2014/124002
- WO-A1-2022/067332
- US-A1- 2009 098 519
- US-A1- 2019 015 751
- US-A1- 2019 019 581
- US-A1- 2019 043 610
- US-A1- 2020 012 959

## Description

### TECHNICAL FIELD

Various embodiments relate generally to systems and methods for delivering regulated digital therapeutic media in a non-regulated media content delivery network.

### BACKGROUND

Medical therapy may include various exercise and/or equipment. Patients may undergo medical therapy under the supervision of a caretaker (e.g., physician, therapist). Medical therapy may, for example, be performed at a medical facility (e.g., hospital, clinic) and/or in a home-based environment.

Medical devices may, for example, include software components. Software components may be regulated by one or more regulatory bodies, such as the Food and Drug Administration in the United States of America, the European Medicines Agency (EMA), the European Food Safety Authority (EFSA), the Veterinary Medicines Directorate (VMD) in the United Kingdom, Health Canada, the Canada Food Inspection Agency (CFIA), the Australian Pesticides and Veterinary Medicines Authority (APVMA), the National Medical Product Administration (NMPA) in the People's Republic of China, the Taiwan Food and Drug Administration (FDA), the Pharmaceutical and Medical Device Agency (PMDA) in Japan, the Ministry of Health, Labor, and Welfare (MHLW) in Japan, and/or the Ministry of Food and Drug Safety (MFDS) in the Republic of Korea. Medical device software components may be required to be tested according to regulatory standards before release.

Non-medical therapy may, for example, include health and/or performance enhancement. For example, various supplements and/or processes may be created to enhance performance and/or health. Such non-medical therapy may be non-regulated or subject to less restrictive legal requirements.

Digital assets may, for example, be available from one or more repositories and/or platforms (e.g., interactive). The digital assets may, for example, be available from game platforms. Digital assets may, for example, be available from software developers. Digital assets may, for example, be available from book publishers and/or retailers. Digital assets may, for example, be available from music and/or video publishers or retailers.

US 2020/012959 A1 discloses a system and method for identifying and leveraging relationships between media consumption and health. It collects data on a user's media usage (e.g., music, videos), health metrics, and contextual factors, synchronizing them in time to analyze correlations. The system uses these analyses to provide personalized media recommendations that support specific health goals, such as reducing stress or enhancing focus.

WO 2014/124002 A1 discloses a system that leverages interactive games in virtual environments to support medical diagnosis and therapy, particularly for pain conditions. Real-time biomedical data is collected during gameplay, processed, and used to optimize treatment. The integration of technologies such as facial recognition, motion tracking, and multisensory approaches enables objective pain assessment and personalized treatment plans. The system is designed for both clinical use and portable applications for home-based care.

US 2009/098519 A1 discloses a method and an apparatus for reward-based learning of policies for managing or controlling a system or plant. In one embodiment, training data consisting of state-action pairs and corresponding rewards is processed to reduce data dimensions, creating a more efficient learning environment. Reward-based learning methods, such as reinforcement learning, are then applied to derive policies for effective system control or optimization. This approach can be applied in various domains, such as managing data centers or controlling physical processes, to enable adaptive and automated decision-making.

### SUMMARY OF THE INVENTION

The invention is set out in the appended claims.

### SUMMARY OF RELATED DISCLOSURE

Insofar as the term embodiment or aspect or alternative is used in the following, or features are presented as being optional, this should be interpreted in such a way that the only protection sought is that of the invention claimed and defined in the appended claims.

Apparatus and associated methods relate to a dynamic inducement package generation system (DIPGS). In an illustrative example, the DIPGS may include at least one validated digital therapeutics (DTx), user profiles, and media profiles associated with wild media content. The DIPGS, for example, may include an assessment engine configured to generate or update a user profile based on a journal of user input and observation of user behavior. The DIPGS may include a medical media package generation engine (MMPGE) configured to generate a dynamic inducement media package (DIMP). In an inducement mode, the DIMP may play the user-selected wild media content regularly. After transitioning from the inducement mode to a therapeutic mode, the MMPGE generates an interventive monitoring content (IMC) comprising a validated DTx to provide therapeutic treatment to the user. Various embodiments may advantageously provide continuous medical guidance and progress monitoring to a user.

Various embodiments may achieve one or more advantages. For example, some embodiments may advantageously provide historical and/or instant assessment of mental and/or physical health of the user. Some embodiments may, for example, advantageously be configured to monitor ongoing media use of the user. For example, some embodiments may include a medical professional interface to advantageously allow professional insight to the user and/or to directly interact with the patient. Some embodiments may, for example, advantageously help self-treatment patients by providing dynamically generated immersive treatment mechanisms based monitoring and tracking mechanisms in an unregulated media content environment. For example, some embodiments may advantageously provide the public with a simple tool to assist in a user's health condition based on user experience regardless of the environment without attachment to a game or a specific media. Some embodiments, for example, may advantageously verify updates on the validated DTx automatically.

The details of various embodiments are set forth in the accompanying drawings and the description below. Other features and advantages will be apparent from the description and drawings, and from the claims.

### BRIEF DESCRIPTION OF THE DRAWINGS

FIG. 1A and FIG. 1B depict an illustrative dynamic inducement package generation system (DIPGS) including a medical media package platform (MMPP) and a dynamic inducement package state machine (DIPSM) in an illustrative use-case scenario.
FIG. 2 is a block diagram depicting an exemplary MMPP.
FIG. 3 is a flowchart illustrating an exemplary runtime method of an exemplary DIPGS.
FIG. 4 depicts an exemplary architecture of an exemplary risk assessment engine of a DIPGS.
FIG. 5 depicts an illustrative use-case scenario of delivering an exemplary independent digital therapeutics (DTx) modality.
FIG. 6 depicts an exemplary digital game cloud (DGC) for delivering DTx games employed in an illustrative use-case scenario.
FIG. 7 is a block diagram depicting an exemplary DGC.
FIG. 8 depicts an exemplary regulatory module to address regulatory requirements for DTx games.
FIG. 9 depicts an exemplary method to validate a change in a DTx.
FIG. 10 depicts an exemplary method of delivering DTx games and generating DTx guidance to a user.

Like reference symbols in the various drawings indicate like elements.

### DETAILED DESCRIPTION OF ILLUSTRATIVE EMBODIMENTS

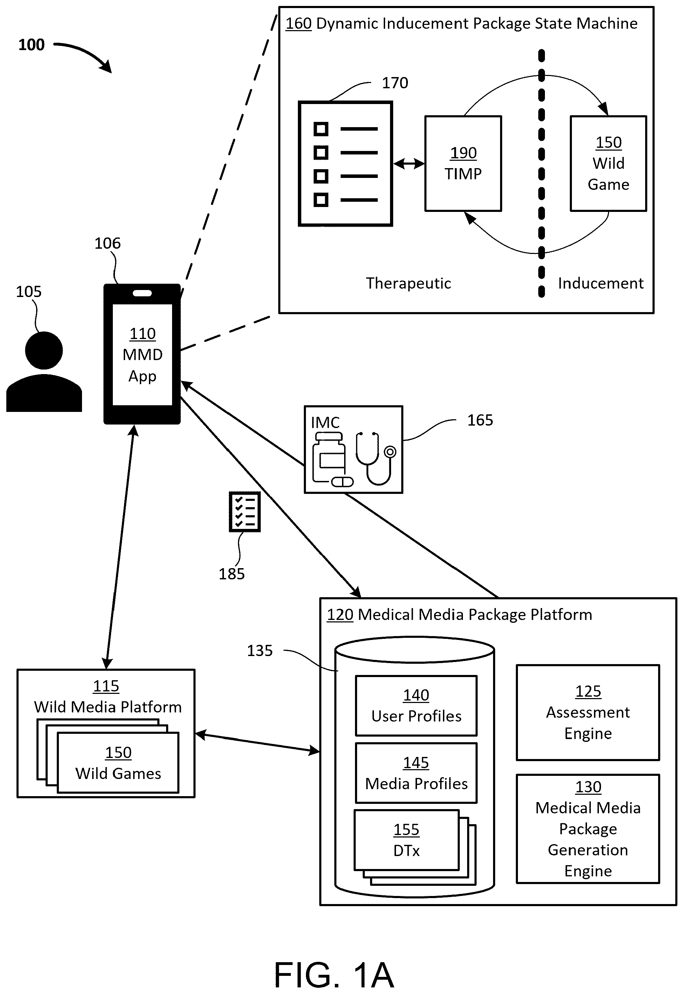

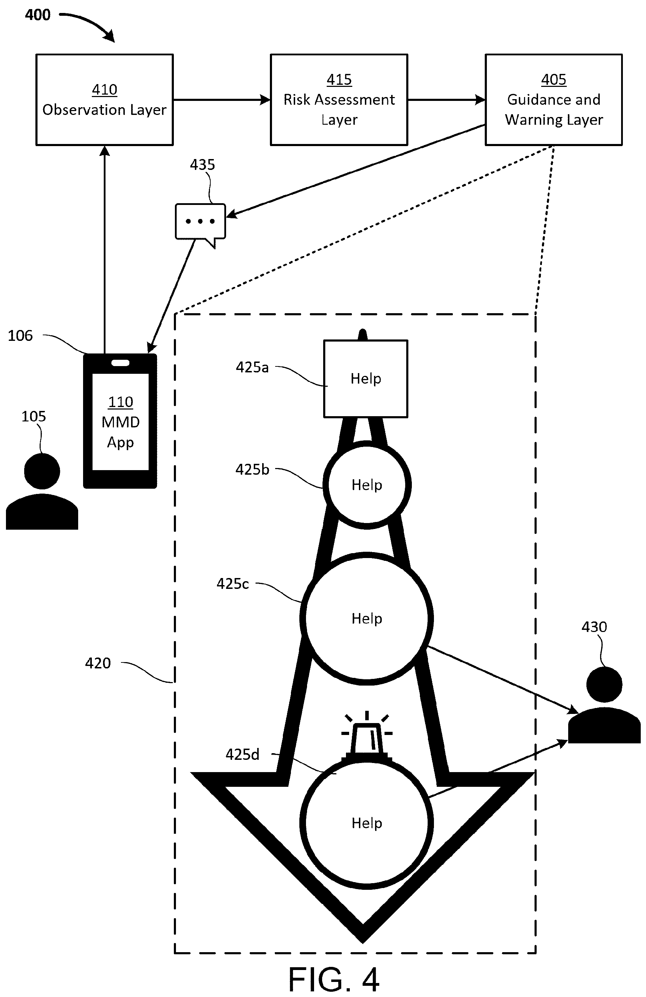

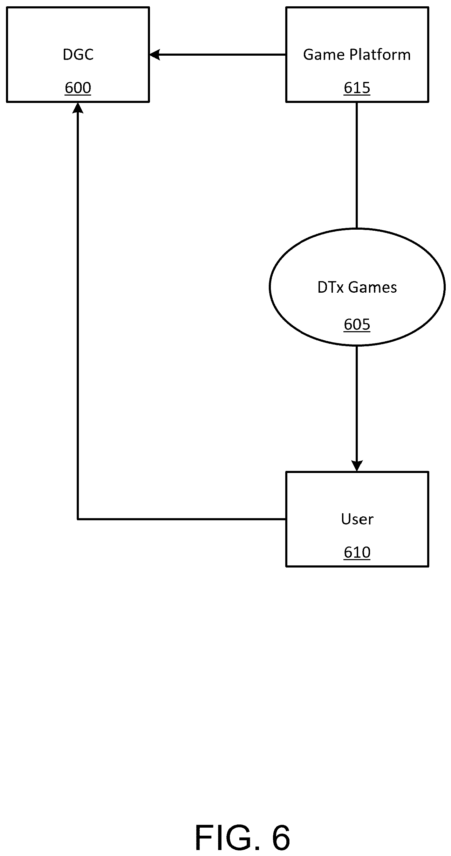

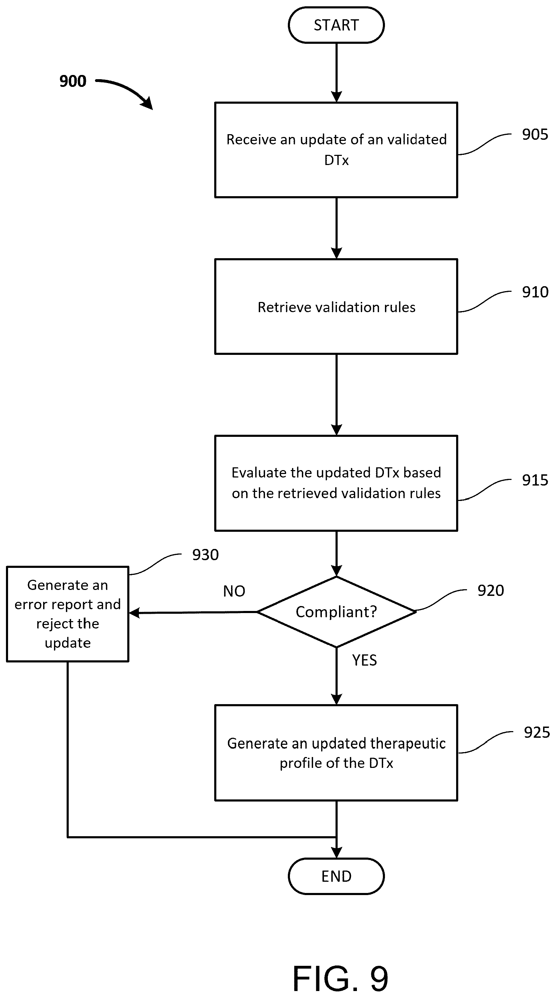

To aid understanding, this document is organized as follows. First, to help introduce discussion of various embodiments, a dynamic inducement package generation system (DIPGS) is introduced with reference to FIGS. 1A-3. Second, that introduction leads into a description with reference to FIGS. 4-5 of some exemplary embodiments of risk assessment and delivery features of the DIPGS. Third, with reference to FIGS. 6-8, a digital game cloud is described in application to exemplary implementation of the DIPGS. Fourth, with reference to FIGS. 9-10, this document describes exemplary apparatus and methods useful for updating and delivering a validated digital therapeutic modality. Finally, the document discusses further embodiments, exemplary applications and aspects relating to dynamic inducement package generations and applications.

Digital therapeutic media, such as audio or video content, may be used to treat various health conditions, including, for example, mental health disorders, chronic pain, and addiction. The delivery of such media, in some examples, may be traditionally restricted to regulated channels, such as regulatory (e.g., US Food and Drug Administration (FDA)) approved telemedicine platforms and/or medical devices, which may limit accessibility and scalability to the patients. Non-regulated media content delivery networks, such as streaming services or social media platforms, may sometimes offer greater reach and flexibility. However, these platforms may, for example, lack the regulatory controls necessary for delivering digital therapeutic media.

In some implementations, digital therapeutic media may be delivered through a variety of means, such as mobile applications, virtual reality platforms, and/or web-based portals. Such platforms may, for example, offer patients a convenient and accessible way to receive treatment, outside of the traditional treatment rooms and independent of their location.

In some examples, digital therapeutic media may be used to train patients to perform involuntary actions (e.g., breathing). For example, these involuntary actions may improve mental health. In an illustrative example, breathing exercises may be an effective treatment for a variety of mental health conditions, including anxiety and depression. Traditional methods of training patients to perform these exercises, for example, are time-consuming and expensive. Sometimes, in a treatment room environment, for example, the patient may feel even more stressed. By using digital therapeutic media, for example, patients may receive training in the comfort of their own homes, at their own pace, and without the need for a therapist or other healthcare professional to be present.

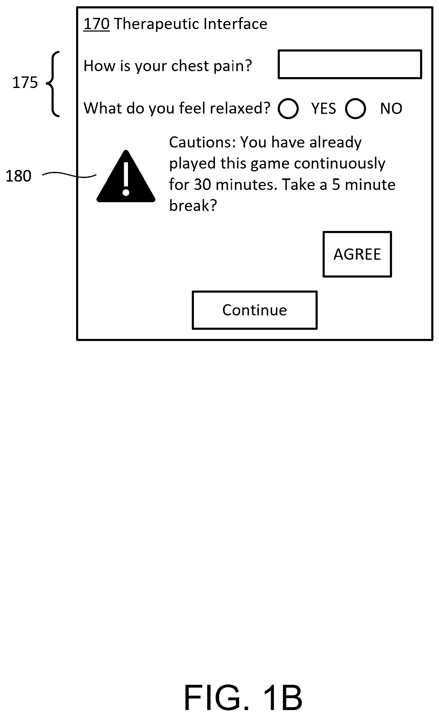

FIG. 1A and FIG. 1B depict an illustrative dynamic inducement package generation system (DIPGS) including a medical media package platform (MMPP) and a dynamic inducement package state machine (DIPSM) in an illustrative use-case scenario. In an example shown in FIG. 1A, a DIPGS 100 includes a self-treating user (STU 105). For example, the STU 105 may be seeking media (e.g., a game) using a device 106 (e.g., tablet, gaming console, personal computer). The device 106 is (e.g., in response to input from the STU 105) running a medical media delivery application (MMDA 110). For example, the MMDA 110 may help the STU 105 to select a game that has therapeutic benefits for the STU 105. For example, the device 106 may be a playing station, a computer, a phone, or some combination thereof.

As shown, the DIPGS 100 includes a wild media platform (WMP 115) and a Medical Media Package Platform (MMPP 120). The MMPP 120, as depicted, includes an assessment engine 125, a medical media package generation engine (MMPGE 130), and a data store 135. The data store 135 may, for example, store input from the STU 105. The data store 135 may, for example, store media information (e.g., a profile of a range of available media). The data store 135 may, for example, store (predetermined) caretaker profile(s).

In the depicted example, the data store 135 includes user profiles 140. The user profiles 140 may, for example, be embodied as digital data structures having predetermined digital metadata associations with corresponding patients (e.g., the STU 105). For example, a user profile may include attributes (e.g., motivational attributes, pathology attributes, dynamic pathology state attributes), and/or temporally-distributed (activity) history of the STU 105. For example, the user profiles 140 may include a player type that may be generated based on an expanded Bartle model.

In the depicted example, the data store 135 includes media profiles 145. For example, the media profiles 145 may be embodied as digital data structures corresponding to specific media objects (e.g., digital media, games, game components). The media profiles 145 may, for example, define predetermined attributes of associated media objects. For example, the MMPP 120 may select and/or filter games suitable for a user based on the predetermined attributes of associated media objects.

The WMP 115 includes various wild games 150. For example, the wild games 150 may be unregulated medically. For example, the wild games 150 may include games, music, and/or videos developed for normal use (e.g., entertainment). For example, the wild games 150 may be uploaded to the WMP 115 by the game developers. In some examples, the DIPGS 100 may include games preloaded to the WMP 115. In some embodiments, the WMP 115 and the MMPP 120 may be in a unified architecture and/or be connected via a communication network. In some embodiments, the WMP 115 may, for example, include other unregulated digital media. For example, the unregulated media may include books. In some embodiments, the unregulated media may include, for example, film. The unregulated media may, in some implementations, include audio, for example. The unregulated media may, for example, act as an attraction mechanism to draw a user (e.g., the STU 105) to continue therapy and/or assessment.

The MMPP 120 may, for example, categorize the STU 105 using the user profiles 140. The MMPP 120 may, for example, use the media profiles 145 to categorize the wild games 150. The MMPP 120may, for example, compare the categorizations and curate a therapeutic journey of the STU 105 through one or more of the wild games 150. For example, the MMPP 120 may suggest a flow of games and treatment. In some examples, the MMPP 120 may allow the patient to individually suggest flows in treatment.

In an illustrative example, the MMPP 120 may, for example, recommend distraction based therapies. For example, if the STU 105 indicates that he/she has been in a scenario that was hard for them to come through, the MMPP 120 may suggest role playing games through corresponding scenarios where the patient is enabled to respond differently and/or control the scenario.

The data store 135 includes a number of digital treatment modules (DTx module 155). For example, the DTx module 155 may include validated therapeutic media content. For example, the DTx module 155 may include therapeutic effects and mechanisms approved by a regulatory organization (e.g., the U.S. Food and Drug Association, the Federal Communication Commission of the United States). In some implementations, the DTx module 155 may be stored in the data store 135 only after being validated by the MMPP 120. Various embodiments for validating and managing the DTx module 155 are described with reference to FIG. 6.

In various embodiments, a DTx module 155 may, for example, include a therapeutic behavior profile, source code, digital media (e.g., audio, video, image, text), interaction sequence (e.g., interaction profile), or some combination thereof. Various embodiments may, for example, include a therapeutic modality, therapeutic mechanic, treatment mechanic, treatment modality, game mechanic, or some combination thereof, such as is described throughout U.S. Application Serial No. 63/202,881, titled "Immersive Digital Therapy," filed by Ryan J. Douglas on June 28, 2021, the entire contents of which are incorporated herein by reference. For example, at least paragraphs [0017-0026] and [0030-0063] discuss various implementations using therapeutic behavior profiles, game implementation profiles, game attribute profiles, therapeutic mechanics, therapeutic modalities, game mechanics, augmentation and/or adaptation management system, and combinations thereof.

In some implementations, the MMPGE 130 may generate a dynamic inducement media package (DIMP) by integrating one of the wild games 150 with one or more of the DTx modules 155 in response to a request of playing a game from the STU 105. For example, each of the media profiles 145 may correspond to one of the wild games 150. The media profiles 145, for example, may include DTx transition points for integrating the wild games 150. In some implementations, the media profiles 145 may also include metadata to, for example, suggest what type of DTx module 155 may be integrated at each of the software insertion points. In some implementations, at the software insertion points, the DTx module 155 may be inserted such that, in runtime, the software would jump to run instructions in the DTx module 155.

As an illustrative example without limitation, the assessment engine 125 may operate on a user input from the STU 105 to determine one or more suggested games for playing. The assessment engine 125 may determine, for example, some games from the wild games 150 based on the user input, the user profiles 140, and the media profiles 145. For example, the suggested games may be displayed on the MMDA 110 for user selection. After one of the suggested games, for example, the MMPGE 130 may generate a DIMP based on the user selection. For example, the DIPGS 100 may deliver the generated DIMP to the device 106 directly from the MMPP 120. For example, the DIMP may be delivered to the device 106 via the WMP 115.

In this example, the MMDA 110 runs a received DIMP on a dynamic inducement package state machine (DIPSM 160). As shown, the DIPSM 160 includes a therapeutic state and an inducement state. In the inducement state, the DIPSM 160 may, for example, run a selected wild game 150. For example, the selected wild game 150 may be run normally. In some examples, the selected wild game 150 may be run with adjusted parameters (e.g., game speed, control mechanisms). For example, the adjusted parameters may be determined based on the user profiles 140 and the media profiles 145.

In the therapeutic state, for example, the DIPSM 160 may interrupt the game play by running a therapeutic immersive medical package (TIMP 190). In some implementations, the MMPGE 130 may, based on the selected wild game 150 and the integrated DTx modules 155, dynamically generate a set of state transition criteria based on the user profile 140 and the media profiles 145. For example, at the state transition, the MMPGE 130 may generate the TIMP 190 based on the DTx module 155, the user profiles 140, and the media profiles 145.

When the transition criteria is reached at an identified instance in the wild game 150, in some implementations, the DIPSM 160 may switch to the therapeutic state and the MMPGE 130 may identify one of the DTx module 155 based on the user profile 140 to generate an interventive monitoring content (IMC 165) to be played in the therapeutic state. For example, the MMDA 110 may run the IMC 165 to display the TIMP 190 . The TIMP 190, for example, may include guidance to the user at the identified instance. For example, the TIMP 190 may include a warning to the user at the identified instance. For example, the TIMP 190 may include a list of questions to the user at the identified instance. For example, the list of questions may be generated to access/track a therapeutic state of the STU 105. In some implementations, the TIMP 190 may be generated based on the user profile140 and the media profiles of the wild games 150.

As shown in this example, in the therapeutic state of the DIPSM 160, the IMC 165 may induce the MMDA 110 to display a therapeutic interface 170. For example, the therapeutic interface 170 may include validated media content to be presented to the STU 105. As an illustrative example without limitation, an exemplary therapeutic interface 170 is shown in FIG. 1B. For example, the therapeutic interface 170 may display a list of questions 175 for the STU 105 to answer. For example, the therapeutic interface 170 may include a guidance 180. For example, the guidance 180 may include cautions, warning, briefing of information (e.g., mental health information, physical health information), or a combination thereof. The list of questions 175, for example, may be generated from the DTx module 155 based on a current progress of the wild game 150 and the user profile 140.

In some implementations, the STU 105 is prompted for response(s) to the therapy. For example, the STU 105 may be asked how they feel (e.g., absolute feeling, feeling relative to just before the media was delivered). The STU 105 may, for example, be asked to provide feedback related to their pathology, motivation, experience, the delivered media, or some combination thereof. Prompts may, for example, include selection (e.g., pick the smiley face or frowny face), ranking, sentence completion, free response, or some combination thereof. In some implementations, additional microjournaling prompts may be provided.

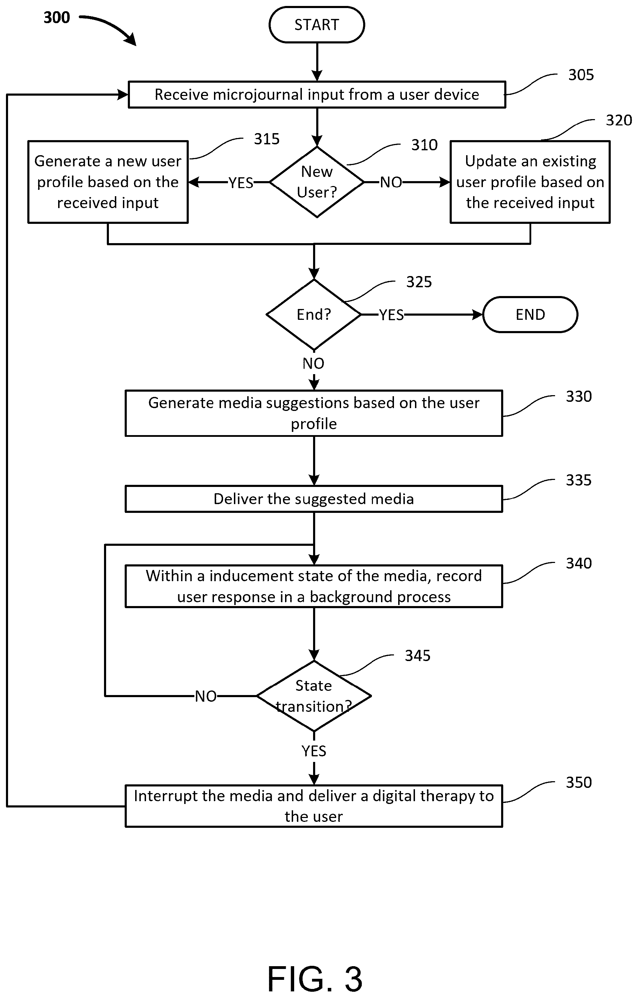

After the STU 105 interacts with the therapeutic interface 170, as shown in FIG. 1A, the MMDA 110 may transmit an answer 185 to the MMPP 120. The assessment engine 125, for example, may process the answer to update the user profiles 140. Various embodiments on updating the user profiles 140 and other exemplary methods for processing the answer 185 are discussed with reference below to FIGS. 3-4.

In an illustrative aspect, the DIPGS 100 including various wild media content (e.g., the wild games 150) may be configured to generate a DIMP based on a user profile generated from a journal of user input. In a runtime of the DIMP, the DIPGS 100 may use the MMPGE 130 to generate a TIMP during playing of a selected media content. For example, the MMPGE 130 may generate the TIMP based on a set of state transition criteria. Based on a received response of the TIMP (e.g., the answer 185), a user profile (e.g., of the user) may be updated.

In some implementations, the MMPGE 130 may generate dynamically the set of state transition criteria based on the selected media content, the user profile, and identified therapeutic instances in the DIMP. In some examples, the generated DIMP may include a recommended set of validated media content (e.g., the DTx modules 155) at each of the identified instances based on the media profiles 145 of the media content (e.g., the wild games 150) and the user profile 140.

In some implementations, the user profiles 140 may be generated based on a journal of user input (e.g., an initial profile of the user, inputs from medical personnel, the answer 185 received during a media playing process) received from the MMDA 110. For example, based on the journal of the user input, the DIPGS 100 may advantageously provide historical and instant assessment of mental and/or physical health of the STU 105.

In various implementations, the IMC 165 may include guidance and warnings to the user presented at a predetermined time based on the set of state transition criteria. For example, the guidance (e.g., the guidance 180) may include instructions to induce autonomic function using voluntary action required in playing the DIMP.

As an illustrative example without limitation, many people may turn to a media as a relief from mental and/or physical health issues. However, such users may lack guidance for which media best suits their issue. Such users may, for example, not have even a subjective measure of whether the media is appropriate for them and/or if their particular condition / situation requires more directed care. Moreover, such users may have difficulty tracking if the media they are using has a positive effect on the condition and/or determining if additional help should be sought.

In various embodiments, the DIPGS 100 may, for example, advantageously be configured to monitor ongoing media use of the STU 105. The DIPGS 100 may, for example, determine (e.g., based on the journal of user input) whether a deterioration in wellbeing during the time media is detected (by the assessment engine 125). In some implementations, the DIPGS 100 may be used to try and address concerns (e.g., within the media use and during the days, weeks, months, and/or years that the individual is interacting with media for specific health concerns). In some implementations, by way of example and not limitation, the DIPGS 100 may be configured as a media delivery therapy companion system. In some implementations, the DIPGS 100 may incorporate a media platform.

In some embodiments, the DIPGS 100 may, for example, be configured as companion code to a (digital) media asset. The DIPGS 100 may, for example, be configured as a wrapper application (e.g., software). The DIPGS 100 may, by way of example and not limitation, be configured as a supplemental app (e.g., mobile app, game console app, the MMDA 110). The DIPGS 100 may, for example, be directly integrated into a media delivery system delivering the media.

In an illustrative example without limitation, if a user requires in-game health assessment, he/she may have to be created by trained medical professionals for medical advice. The trained medical professionals may be required to, before providing any medical advice, study a set of media (e.g., games, songs, movies). After all the media are studied thoroughly (e.g., watching a 10-minute video for a few times, studying the transcript of the video), the medical profession may identify suitable interruption points and create guidance at each of the interruption points based on a detailed medical record associated with the user. Accordingly, the medical profession would easily spend days, if not weeks, just to prepare a professional program of one media for one user.

To provide self-assessment, the user would have to interrupt himself at the identified time. This would be very difficult and only a disciplined user may be able to do. On the other hand, the user may play the media accompanied by a medical professional to be interrupted during playing, and to deliver the medical guidance to the user. In this way, the cost of playing the media would be prohibitive. Furthermore, after the user is bored with the media with the professional program, expanding the library of media would require the same costly process.

Using the DIPGS 100, the STU 105 may automatically enjoy validated media content generated by the MMPGE 130. The assessment engine 125, for example, may advantageously provide medical guidance and monitor the progress of the STU 105. In some implementations, as described with reference to FIGS. 6-9, the media content delivered to the STU 105 may be regulated. For example, the delivered content may be compliant to regulatory rules.

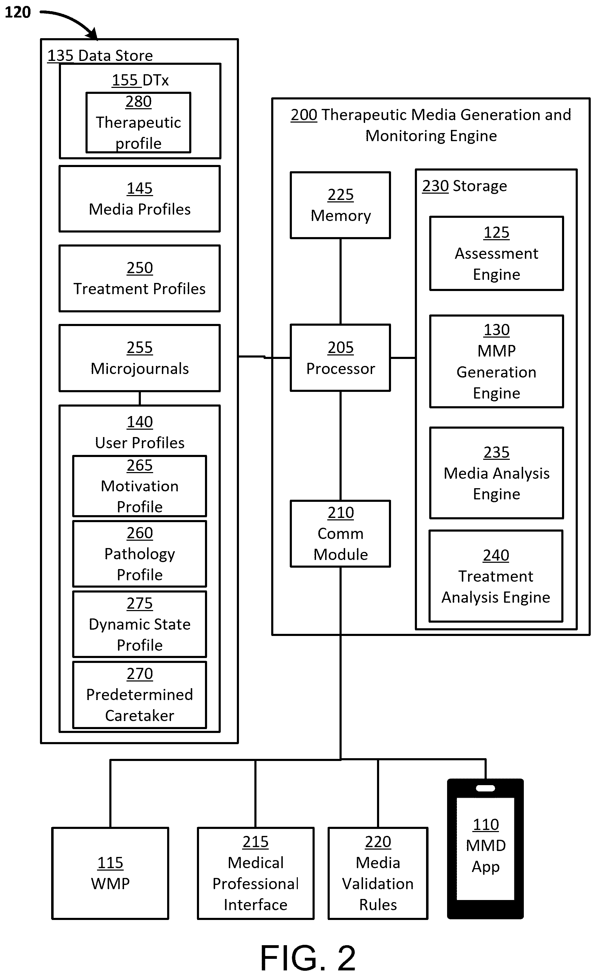

FIG. 2 is a block diagram depicting an exemplary MMPP (e.g., the MMPP 120). In this example, the MMPP 120 includes a therapeutic media generation and monitoring engine TMGAME 200. The engine MGAME 200 includes a processor 205. The processor 205 may, for example, include one or more processors. The processor 205 is operably coupled to a communication module 210. The communication module 210 may, for example, include wired communication. The communication module 210 may, for example, include wireless communication. In the depicted example, the communication module 210 is operably coupled to the MMDA 110 and the WMP 115. In the depicted example, the communication module 210 is operably coupled to a medical professional interface 215 and medical validation rules 220. For example, the medical professional interface 215 may allow medical professionals to adjust data in stored in the MMPP 120. For example, the medical professional interface 215 may allow medical professionals to provide medical input to the MMPP 120. The medical professional interface 215, for example, provides a communication channel(s) between the medical professional and the patient. For example, the medical professional may, via the medical professional interface 215, ask, "How did you feel about that game? That game happens to be set up around the seven stages of grief." Accordingly, the medical professional may advantageously provide insight to the patient and/or may directly interact with the patient.

In some implementations, the DIPGS 100 may allow the medical professional to suggest flows in treatment and/or 'homework' to a user. For example, the medical professional may indicate that the MMPP 120 to heavily focus on anxiety for a while for a specified user. For example, the medical professional may indicate that a traumatic issue will be addressed later after anxiety is addressed.

The memory module 225, for example, may be used to validate media content transmitted by the MMPP 120. For example, the MMPP 120 may use the memory module 225 to automatically verify a generated IMC or DIMP.

The processor 205 is operably coupled to a memory module 225. The memory module 225 may, for example, include one or more memory modules (e.g., random-access memory (RAM)). The processor 205 includes a storage module 230. The storage module 230 may, for example, include one or more storage modules (e.g., non-volatile memory). In the depicted example, the storage module 230 includes the assessment engine 125 and the MMPGE 130.

The MMPGE 130 may, for example, determine an interface 170 to cause to be generated and displayed to the STU 105. The assessment engine 125 may, for example, based on input from the user, update the user profiles 140.

The media analysis engine 235 may, for example, analyze media objects (e.g., the wild games 150, other media content associated with the WMP 115) to determine attributes. For example, in some implementations, the media analysis engine 235 may be configured to generate the media profiles 145. In some implementations, the media analysis engine 235 may include machine learning model(s) configured to automatically redefine and/or update weightings associated with attributes of a media object based on user input (e.g., microjournaling, changes in pathology profiles, caretaker input, authorized expert input).

In the depicted example, the storage module 230 includes a treatment analysis engine 240. For example, the treatment analysis engine 240 may generate output based on data stored in the data store 135. The data store 135 is operably coupled to the processor 205. The data store 135, as depicted, includes the media profiles 145, the user profiles 140, a treatment profile 250, and microjournals 255.

The microjournals 255 may, for example, be embodied as digital data structures having a predetermined digital metadata association to a specific user (e.g., to a user profile 140). The microjournals 255 may, by way of example and not limitation, store historical responses (e.g., raw, summarized, assessments of raw responses) of a corresponding user to therapy (e.g., to media presented).

In various embodiments, the microjournals 255 may include, by way of example and not limitation pre and post game play inputs, with associated database that correlates games played, duration, frequency, and/or emotional state as represented by user-entered and/or system-deduced information. Such information may, by way of example and not limitation, include rating scales of emotion (e.g., 1-5, emoticons), word association, sentence completion (e.g., related to current symptom state), free writing inputs, gratitude journals, experience journals, or a combination thereof.

The user profiles 140 includes a pathology profile 260, a motivation profile 265, a predetermined caretaker 270, and a dynamic state profile 275. The motivation profiles 265 may, for example, be embodied as digital data structures having a predetermined digital metadata association to predetermined motivational types. The motivation profiles 265 may, for example, define attributes of motivational types.

For example, the motivation profiles 265 may include one or more profiles based on the above model. A motivation analysis engine 240 may generate, for example, the motivation profile 265 for a user as a weighted distribution of multiple motivational types.

Illustrative examples of self-assessments that may be implemented to help determine if a user is suffering from symptoms of mild to moderate stress, anxiety, and/or depression may include but are not limited to the Patient Health Questionnaire (PHQ-9). The PHQ-9 may, for example, be a validated screening measure that aids in the diagnosis and assessment of severity of depressive symptoms and may, for example, serve as a symptom severity tracker to monitor effectiveness of a treatment plan. In some implementations, by way of example and not limitation, a MMPP 120 may be configured to use validated assessment measures to better 'understand' core symptoms of each disorder/symptom state (e.g., generate the pathology profiles 260, generate the treatment profiles 250). In depression, for example, SIGECAPS (a mnemonic device that outlines the pathognomonic symptoms of depression as listed in the Diagnostic and Statistical Manual of Mental Disorders, Fifth Edition (DSM-5)) may be implemented as a framework.

The treatment profiles 250 may, for example, be embodied as digital data structures corresponding to specific therapies. One or more treatment profiles 250 may, for example, be linked to one or more patient pathology profiles 260, and/or vice versa. A treatment profile 250 may, by way of example and not limitation, define predetermined attributes of therapy (e.g., breathing profile, time durations, indicated symptoms, indicated pathologies).

In some implementations, the treatment analysis engine 240 may, by way of example and not limitation, be configured to operate on the user profiles 140 to determine associations of a user to one or more pathologies. For example, the treatment analysis engine 240 may generate the pathology profile 260 based on data associated with and/or retrieved from one of the user profiles 140. For example, the pathology profile 260 may be generated corresponding to attributes defined by one or more pathology definitions. For example, the treatment analysis engine 240 may generate the pathology profile 260 including one or more weightings associated with each of multiple pathology definitions.

For example, the pathology definitions may, for example, be embodied as digital data structures corresponding to specific pathologies (e.g., depression, cardiomyopathy, post-traumatic stress disorder). A pathology definition may, for example, define predetermined attributes (e.g., associated symptoms, associated therapies) of a corresponding pathology. In some implementations, the pathology definitions may define one or more states of a pathology and associated attributes. In some implementations, the pathology definitions may be accessible in an external database using the communication module 210. In some implementations, the pathology definitions may be pre-loaded in the data store 135.

In this example, the data store 135 also includes the DTx module 155. As shown, each of the DTx module 155 may include a therapeutic profile 280. For example, the therapeutic profile 280 may include attributes and parameters of the corresponding DTx module 155. For example, the MMPGE 130 may generate the DIMP from a DTx module based on the therapeutic profile 280.

In some implementations, the treatment analysis engine 240 may, for example, operate on user profiles 140 (e.g., the patient pathology profile 260 retrieved from and/or by association with a user profile(s)) as a function of the treatment profiles 250 to generate an associated patient treatment profile(s) (not shown). For example, the treatment analysis engine 240 may, for example, determine suggested media content based on the user profiles 140 as a function of the treatment profiles 250, the media profiles 145, and/or a motivation profile 265 of the user profile 140. The treatment analysis engine 240 may, for example, include machine learning model(s) configured to automatically redefine and/or update weightings associated with attributes of a patient treatment profile based on user input (e.g., microjournaling, changes in pathology profiles, changes in media profiles, caretaker input, authorized expert input).

In some implementations, the assessment engine 125 may, for example, operate on user profiles 140 and/or the microjournals 255 to determine a current state of associated user(s). In some implementations, by way of example and not limitation, the assessment engine 125 may operate on a user profile 140 and/or microjournal 255 associated with a patient (e.g., the STU 105) to determine a current state(s) of the user (e.g., in a pathology process) as a function of the treatment profiles 250. In some implementations, the assessment engine 125 may, for example, operate on user profiles 140 and/or microjournals 255 to determine a current state of associated user(s). In some implementations, by way of example and not limitation, the assessment engine 125 may operate on a user profile 140 associated with a patient (e.g., the STU 105) and/or motivation profiles 265 to determine the motivational profile 265 of the user.

In some implementations, the assessment engine 125 may generate a message to the medical professional interface 215 and/or the predetermined caretaker 270 when it is determined that an in-person intervention is required. For example, the assessment engine 125 may be configured, by way of example and not limitation, to generate the dynamic state profile 275 for a user using, for example, a Sleep, Interest, Guilt, Energy, Concentration, and Appetite, Psychomotor, and Suicidal ideation (SIGECAPS) framework to access a present mental health state of the user. For example, the assessment engine 125 may determine to contact the predetermined caretaker 270 based on an updated dynamic state profile 275

FIG. 3 is a flowchart illustrating an exemplary runtime method 300 of an exemplary DIPGS. For example, the MMPP 120 may be used to perform the method 300. The method 300 begins in step 305 when an input is received from a user device. For example, the device 106 may transmit an input from the STU 105 to the MMPP 120. In some embodiments, an MMPP 120 may include an ongoing evaluation engine configured to help determine, by way of example and not limitation, an effectiveness of a game, a list of recommended games, whether the user should be directed to greater levels of personal care (e.g., instead of self-treatment).

For example, the STU 105 may use the MMDA 110 to send a user input including a microjournal description of self-assessment to the MMPP 120. In a decision point 310, it is determined whether the user is a new user. If the user is a new user, in step 315, a new user profile is generated based on the received input. For example, the new user profile may be stored in the user profiles 140. If the user is not a new user, in step 320, an existing user profile is updated based on the received input. For example, the assessment engine 125 may update the microjournals 255 in the user profiles 140 based on the user input.

For example, the input operated on in the step 305 may include responses to a predetermined question set presented to the STU 105. In some implementations, the question set may be retrieved from the data store 135 and presented to the STU 105. In some implementations, the question set may be dynamically generated based, for example, on responses from the STU 105. In some implementations, the question set may be provided to the STU 105 by a third party (e.g., the STU 105 may be directed to the question set and/or signals representing results from a third-party question set may be transmitted to the MMPP 120).

After the step 315 or the step 320, in a decision point 325, it is determined whether the method 300 is to be ended. For example, the STU 105 may close the MMDA 110 after completing microjournaling. If the method 300 is to be ended, the method 300 ends.

If the method 300 is not to be ended, media suggestions are generated based on the user profile in step 330. For example, the MMPGE 130 may generate a list of suggested wild games 150 to the user based on the user profile 140 of the user. Next, in step 335, the suggested media are delivered. In some implementations the media may, for example, be delivered from and/or via the WMP 115. For example, the MMPP 120 may deliver the link to the suggested media in the WMP 115 to the STU 105. For example, the MMPP 120 may deliver the DIMP containing the suggested media to the STU 105. For example, the media analysis engine 235 may operate on the user profile 140 to generate suggested media as a function, by way of example and not limitation, of media profiles 145, pathology profiles 260, treatment profiles treatment profiles 250, motivation profiles 265, the assessment engine 125, the patient pathology profile 260, and/or the dynamic state profile 275.

In step 340, user responses are recorded in a background process within an induced state of the media. For example, the DIPSM 160 may transmit the user profiles 140 based on user's interaction within the wild games 150 in the inducement state. The response may, for example, be transmitted periodically. The response may, for example, be transmitted when a predetermined criterion is met.

In a decision point 345, it is determined whether a state transition is to be performed. For example, the DIPSM 160 may determine that a transition from the inducement state to the therapeutic state is to be performed when a set of predetermined criteria generated by the MMPGE 130 is met. If a state transition is not to be performed, the step 340 is repeated. If a state transition is to be performed, in step 350, the media is interrupted and a digital therapy (e.g., the TIMP 190) is delivered to the user (e.g., to the MMDA 110), and the step 305 is repeated. For example, the DTx module 155 may, by way of example and not limitation, include therapeutic suggestions and/or instructions (e.g., such as cautions via the therapeutic interface 170 as disclosed at least with reference to FIG. 1B). For example, responses of the STU 105, history of media suggestions and/or selected media, history of interaction(s) with the delivered media, or some combination thereof may, for example, be saved in association with the user profile 140 (e.g., in a microjournal 255).

Ism, after the step 320, the user profiles 140 of other users be updated (e.g., media profiles 145, pathology profiles 260, treatment profiles 250, motivation profiles 265). For example, the user profile 140 may be updated based on actual actions and/or responses of a similar user to increase accuracy instead of relying solely on an initial self-assessment.

In various implementations, the DIPSM 160 configured to, during the runtime of the DIPGS, identify one of the therapeutic instances in the DIPGS, interrupt the runtime of the DIPGS, presents one or more therapeutic modality to the user, and update the user profile and the DIPGS based on a result of the therapeutic modality.

FIG. 4 depicts an exemplary architecture 400 of an exemplary risk assessment engine of a DIPGS. In the depicted example, a guidance and warning layer 405 may, for example, provide media and/or prompts to a user and/or receive input from the user. An observation layer 410 may, for example, generate metrics corresponding to predetermined criteria based on input from the guidance and warning layer 405. A risk assessment layer 415 may generate indications and/or messages corresponding to referral based on the metrics. In some implementations, the guidance and warning layer 405, the observation layer 410, and the risk assessment layer 415 may be included in the assessment engine 125.

As shown in an illustrative example in FIG. 4 without limitation, the STU 105 may, for example, be self-treating using media in the WMP 115. The observation layer 410 may receive input (e.g., via a display layer module) corresponding to use of the media by the STU 105 and/or progress related to a monitored condition.

As an illustrative example, the observation layer 410 may be configured to collect information (e.g., prompt the user to input a rating) about a health metric. For example, a user may be seeking to self-treat pain and/or range of motion restrictions. The user may, for example, rate pain and/or range of motion. Games may be suggested and/or delivered that induce the user to incrementally move in a way that may address the pain and/or range of motion limitation. The observation engine may prompt the user to provide periodic feedback. Based on the feedback, an assessment engine may make recommendations (e.g., continue playing, play another game, seek further assistance). For example, a 'referral' recommendation may be generated based on predetermined metrics (e.g., a pain scale and/or an increase in pain metric exceeding a predetermined referral criterion).

Accordingly, the observation layer 410 may advantageously help users be more aware of predetermined factors (e.g., pain, depression, etc.). In some implementations, the observation layer 410 may observe a user's behavior (e.g., during game play, out of a game play) to collect health metric information. For example, based on the collected information, the observation layer 410 may solicit responses (e.g., suggests a game, transmit a questionnaire) to the user. For example, in an illustrative use case regarding depression, if a user ranks their feelings corresponding to mild/moderate depression, and the user is feeling better after playing, a suggestion layer and/or engine may generate a message recommending to play tomorrow. If the user is feeling worse, a message may be generated recommending the user to play different games. If the user indicates additional stressors and/or significant deterioration, a message and/or indication may be generated prompting the user to seek help (e.g., a predetermined caretaker).

In some embodiments, an observation layer and/or engine may monitor physical attributes of a user. For example, physical monitoring may include biological sensors (e.g., heart rate, temperature, blood pressure). In some embodiments, physical monitoring may, for example, include a camera(s). For example, the camera may be configured to detect posture. In some embodiments, metrics may include response speed(s) (e.g., during playing a game). Recommendations may, for example, be determined based on physical attributes.

The risk assessment layer 415 may operate on metrics determined by the observation layer 410 from the input. The risk assessment layer 415 may compare the metrics to predetermined criterion(s) relating to progress and/or risk. The risk assessment layer 415 may generate a signal(s) to change a visual indicia presented to the STU 105 as a function of the metrics and the predetermined criterion(s) (e.g., a risk scale). The guidance and warning layer 405 may, for example, cause (dynamic) indicia 435 to be generated in response to the signal(s) from the risk assessment layer 415. For example, the indicia may be displayed in the therapeutic interface 170 as shown in FIGS. 1A-B.

As an illustrative example, the risk assessment layer 415 may include a risk assessment and/or notification engine configured to generate alerts, remind, and/or direct a player to seek additional care during the course of attempting to use games as a coping mechanism. For example, by way of example and not limitation, the player may be directed to seek additional help when the player is self-assessing beyond a mild to moderate level of symptoms, the player's mood shifts in a negative direction while playing the games, or the player's mood trends to more negative over a duration where relief or at least stability should be expected. In some implementations, the assessment engine 125 may be configured to further help direct the individual to an appropriate resource for their health concerns (e.g., mental, physical) based on one or more factors (e.g., including self-assessment, interactions with the self-assessment and gaming system).

In this example, the STU 105 is presented with a visual indicia 420. In some implementations, the indicia 420 may include a help button. For example, the STU 105 may use the help button to self-identify help. For example, the STU 105 may click on the help button to escalate awareness of potential problems to a caretaker 430 (e.g., a predetermined caretaker, a suggested caretaker). In some implementations, the help button may change in shape and/or color based on risk intensity. For example, when the risk level is low, a help button 425a may be grey and rectangular in shape, as described in the depicted example. When the risk level increases, for example, the help button 425a may change to a help button 425b from grey to green, and its shape may change from rectangle to a circle. The help button 425b, in this example, may increase in size and change color to yellow as shown in a help button 425c when the risk level is further increased to medium-high risk, for example. When the risk level is high, as an illustrative example, a help button 425d may grow further in size, the text may change to red, and may be flashing.

In various embodiments the assessment engine 125 may include a safety mechanism configured such that that in all cases where the media does not or does not continue to serve the individual in addressing their health condition, a referral is made to a predetermined caretaker 430 (e.g., as stored in the predetermined caretaker 270 in the data store 135). The predetermined caretaker 430 may, for example, include a family member. The predetermined caretaker 430 may, for example, include a professional service or other similar help line. This referral may, for example, help the individual self-assess and/or recognize that greater levels of care are required. The referral may, for example, provide a suggested access point for the care.

In various implementations, the media may advantageously act to engage and keep the individual assessing their level of physical / mental health and on a path of self-discovery that may lead to seeking additional care. In an illustrative example, video gamers and/or other people with mental health conditions may be capable of accessing information online, through peer networks, or self-discovery that videogames can be a source of relief from the symptoms of mental health. However, in making this self-determination, selecting and playing these games, they may not choose the right or best fit for themselves. Additionally, use of video gaming for a specific condition may not be warranted and/or may cause additional harm if the individual does not find the relief they are seeking. The very act of self-determining a need for support or care is an important step may, for example, be an on-ramping process for receiving care, especially for those who do not and/or cannot afford care.

In some examples a condition may exist where, once an individual has self-identified as having a mental health concern, they may not be motivated to continue to self-monitor and assess to help determine if the chosen game is helping their condition or if the condition in general, outside the gaming experience, is becoming more severe.

Advising against using videogames to address mental health conditions may remove an important option as a home-based coping mechanism and does not represent a realistic approach to dealing with the current mental health support and treatment environment or how individuals will continue to view games and other sources of media as aids to health and personal improvement. Accordingly, the method 300 may advantageously solve a technical problem for helping self-treatment patients by providing dynamically generated immersive treatment mechanisms based monitoring and tracking mechanisms in an unregulated media content environment for the STU 105.

In some implementations, the DIPSM 160 may be configured to adjust a set of predetermined runtime parameters of the DIMP based on the TIMP 190 and the therapeutic profile of the DTx module 155 in the TIMP 190 and the user profile 140.

As an illustrative example, a patient may download the MMDA 110. The MMDA 110 may prompt the patient, upon registration, to answer some questions. For example, in response to receiving the user input, the assessment engine 125 may determine, in response to the questions, that the patient is not feeling good (e.g., feeling low, feeling bad). The assessment engine 125 may determine that the patient is associated with post-traumatic stress disorder, anxiety, and/or trauma. For example, the assessment engine 125 may determine (e.g., automatically, based on input from a therapist) that the patient is feeling depressed due to trauma and/or that the depression is so linked to trauma that the trauma must be dealt with before other issues.

The assessment engine 125 may determine that a distracting exercise is needed (e.g., based on input from the patient and/or a therapist, based on historical results from other patients with statistically similar responses within a predetermined confidence interval). The MMPP 120 may recommend a tetris-based game ("tetris"). The patient may acquire (e.g., buy, download) tetris (e.g., on the WMP 115, purchase a stand-alone game). The patient may log into the tetris game in a way that notifies the assessment engine 125 accessed to tetris is completed.

In response to the notification, the MMPP 120 may cause the MMDA 110 to generate an interface with cautions and warnings (e.g., as mandated by FDA regulations). The MMPP 120 may cause the app to generate an interface (e.g., "From the data you provided, I want you to visualize the time in which you had the traumatic event and what it was that was scaring you about it."). The DIPGS 100 may, for example, then prompt the user to play tetris (e.g., "Try to play tetris for 20 minutes"). After the suggested amount of time and/or when the MMPP 120 is notified that the user has stopped playing, the MMPP 120 may cause the app to generate an interface (e.g., the therapeutic interface 170) asking the patient one or more questions (e.g., 1-2 brief questions) about how they're feeling now. The MMPP 120 may suggest subsequent actions (e.g., "You should do this again tomorrow").

Accordingly, the DIPGS 100 may use playing tetris, by way of example and not limitation, to induce the patient to reassociate their thoughts that were associated with the trauma. The DIPGS 100 may, for example, not change the game at all from a 'normal' non-medical implementation. The DIPGS 100 may use tetris as a tool in medical therapy by suggesting that the patient be thinking about something while they play it.

The DIPGS 100 may, for example, be collecting and/or analyzing monitoring and/or feedback data to understand why the patient is depressed. The DIPGS 100 may, for example, be sequencing the patient through a group of games that can help the patient treat that depression. The DIPGS 100 may, for example, be adding therapeutic elements to those games (e.g., labeling, suggestions). The DIPGS 100 may, for example, be tracking the patient's behavior over time (e.g., giving the patient and/or physician feedback to gain insight into how well the patient is doing and/or how well the therapy is working) in the user profiles 140.

In some embodiments, the DIPGS 100 may, for example, be configured to enable the patient to perform self-directed care. In the illustration above, for example, if the patient responds that the patient doesn't want to play tetris, the MMPP 120 may suggest other puzzle games. If the patient doesn't want to do puzzle games, but prefers an action game, the MMPP 120 may suggest an action game.

For example, the patient may play the action game. Once the patient has gotten their anxiety a little bit in check, the assessment engine 125 may come back again and suggest that the patient tries to think about these traumatic moments for a minute and go play some tetris. The patient may play for a period of time and then indicate they are tired of playing tetris. The assessment engine 125 may encourage them and suggest another puzzle game while performing therapeutic actions (e.g., thinking about the trauma first). In some such examples, the games may be unchanged relative to an 'off-the-shelf' non-therapeutic use. In some embodiments, a kernel may be provided (e.g., a check-in module).

In some implementations, the guidance and warning layer 405 may further include General Wellness software functions to promote, track, and/or encourage choices, which, as part of a healthy lifestyle, may help living well with or reduce the risk of certain chronic psychiatric diseases or conditions where the link between living well and reducing the risk or impact of a chronic psychiatric disease or medical condition is well understood. For example, chronic psychiatric conditions may include Depression, Anxiety, Obsessive Compulsive Disorder, Autism, Attention Deficit Hyperactive Disorder. The platform may also provide physical wellness and general health treatments and guidelines.

In some examples, the General Wellness software functions may provide statements such as follows, "Device X provides daily motivational reminders to perform physical activity, which may help patients with chronic depression live well," or "Device Y provides mindfulness and meditation activities, which may help patients with chronic anxiety live well."

In some implementations, the General Wellness software functions may help patients with diagnosed psychiatric conditions maintain their Contains Nonbinding Recommendations 12 behavioral coping skills by providing a "Skill of the Day" behavioral technique or audio messages that the user can access when experiencing increased anxiety related to the COVID-19 public health emergency.

In some implementations, the General Wellness software functions may help teach users to "just notice," accept, and embrace difficult or previously unwanted thoughts and feelings during the COVID-19 public health emergency, so that the users open up to these unpleasant feelings and learn not to overreact to them or avoid situations where they are invoked. In some examples, the General Wellness software functions may display, at opportune times, images or other messages for a substance user who wants to stop addictive behavior due to increased anxiety during the COVID-19 public health emergency.

In some examples, the General Wellness software functions may help patients or users self-manage their disease or conditions without providing specific treatment or treatment suggestions. In some examples, the General Wellness software functions may use a checklist or a questionnaire of common signs and symptoms for a psychiatric disorder (e.g., anxiety due to stay-in-place orders) and to provide a list of possible medical conditions and advice on when to consult a health care provider. In some examples, the General Wellness software functions may guide a user through a questionnaire of signs and symptoms for a psychiatric disorder (e.g., anxiety or stress due to stay-in-place orders) and to provide a recommendation for the type of health care facility most appropriate to their needs.

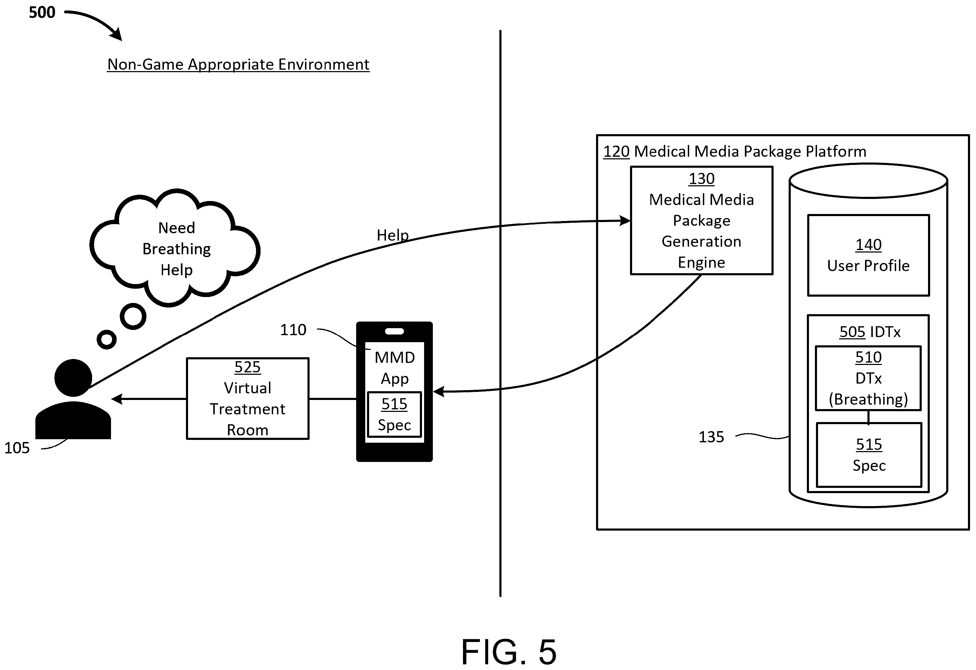

FIG. 5 depicts an illustrative use-case scenario of delivering an exemplary independent digital therapeutics (IDTx) modality. In this example, the STU 105 may be in a non-game appropriate environment 500. For example, the STU 105 may be working in an office and cannot play a game. However, as shown in FIG. 5, the STU 105 may desire to get breathing help. In this example, the STU 105 may transmit, using the MMDA 110, a help signal specifying a help is needed. For example, the help signal may indicate the STU 105 needs breathing help. For example, the help signal may indicate the STU 105 is having an anxiety attack.

After receiving the help signal, the MMPGE 130 may process the help signal to determine, for example, that presentation of the DIMP is not suitable based on environment limitations. In some implementations, the MMPGE 130 may determine the non-game appropriate environment 500 based on the user profiles 140 and a location of the STU 105.

In this example, the MMPGE 130 determines to generate a breathing related IDTx 505 to be delivered based on the user profile 140 of the STU 105. For example, the breathing related IDTx 505 may be a streamlined DIMP. For example, the MMPGE 130 may access the microjournals 255, the motivation profiles 265, and the dynamic state profile 275. For example, the MMPGE 130 may determine the breathing related IDTx 505 based on a historical user feedback and a historical game play of the STU 105 and/or similar user.

As shown, the breathing related IDTx 505 includes a breathing DTx 510 and a specification 515. For example, the breathing DTx 510 may be generated based on previously played TIMP by the STU 105. For example, the specification 515 may be dynamically generated based on the breathing DTx 510 and the user profile 140. In some implementations, the specification 515 may include instructions and warnings to guide the user. In this example, the specification 515 may include guidance for the user in a breathing exercise. For example, the guidance may be generated by artificial intelligence based on the breathing DTx 510, the user profiles 140, and the media profiles 145.

Upon receiving the specification 515, the MMDA 110 generates a virtual treatment room 525. In some implementations, the virtual treatment room 525 may provide a short duration immersive therapeutic experience for the user. As an illustrative example, the virtual treatment room 525 may provide a 3-minute breathing exercise to the user accompanied with anti-anxiety music and/or video.

In various implementations, when the DIPGS 100 determines that presentation of the DIMP is not suitable based on environment limitations, or based on receiving a user request, the DIPGS 100 may generate a streamlined DIMP comprising the TIMP 190 based on the user profile and a history of played DIMPs. Accordingly, the DIPGS 100 may advantageously, for example, provide a solution to provide the public a simple tool to assist in a user's health condition based on user experience regardless of the environment without attachment to a game or a specific media. For example, the DIPGS 100 may act as an 'assistant' for health self-assessment (e.g., physical, mental).

FIG. 6 depicts an exemplary digital game cloud (DGC 600) for delivering DTx games employed in an illustrative use-case scenario. For example, a user 610 may receive the DTx games 605 from a game platform 615.

In some implementations, the DGC 600 may include the MMPP 120 to advantageously enable an integrated platform for individualization, planning, coordination, tracking, and/or delivery of videogames as digital therapeutics. For example, the DTx game 605 may include a DIMP generated by the MMPGE 130. The DGC 600 may, for example, work with one or more supported game platforms (e.g., the WMP 115). The DGC 600 may, for example, enable the user 610 to play games in the games' native location. The DGC 600 may, for example, remove regulatory burden from the individual games, allowing the games to be played as originally intended. For example, the DGC 600 may advantageously perform required regulatory work (e.g., all the regulatory work) to ensure safe and effective delivery of mental health treatment for games delivered using the DGC 600.

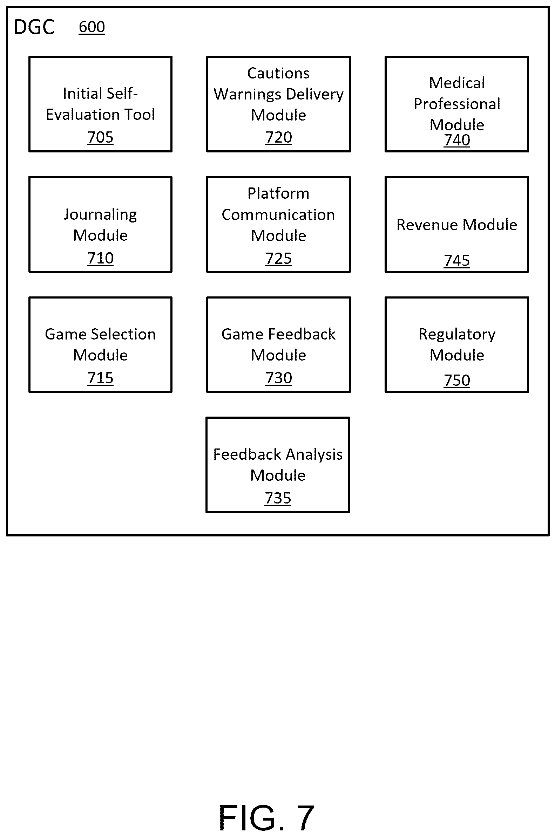

As discuss further with reference to FIGS. 7-8, the DGC 600 may manage regulatory burden by, for example, delivering games that already are cleared as medical devices, delivering games that make wellness claims is association with the clear games, and/or delivering games where the DGC 600 does the regulatory work, including but not limited to labeling, therapeutic setup and/or follow up (preamble and/or post game play messaging, monitoring, or inquiry).

In various implementations, the DGC 600 may receive usage information from the game platform 615. For example, the DGC 600 may identify, from data provided from the game platform 615 that the user 610 is playing a specific game at a particular time. In some implementations, an interconnectivity between the game platform 615 and the DGC 600 may be provided by incorporating a digital connection between wild games of the game platform 615 and the DGC 600. For example, in some implementations the DGC 600 may, at a minimum, be provided a signal identifying a user playing which game and when. In some implementations, when such connection may be absent, the DGC 600 may allow the user 610 to manually report usage information (e.g., use of the game and duration of play).

FIG. 7 is a block diagram depicting an exemplary DGC. In this example, the DGC 600 includes an initial self-evaluation tool 705 and a journaling module 710. For example, the initial self-evaluation tool 705 may help the user 610 determine which games are best suited for their health concern and current state of dysfunction. For example, the initial self-evaluation tool 705 may use the assessment engine 125 to assess the user's current state to recommend the best suited games.

In some implementations, the initial self-evaluation tool 705 may include a point of entry self-assessment tool configured to help a user determine if the DGC is suited for the players' needs. For example, the entry self-assessment tool may determine (e.g., based on profiles in the data store 135) if currently cleared platform indication(s) for use (e.g., predetermined clearance indications associated with pathology profiles, treatment profiles, and/or dynamic pathology states) match the user's needs and current profile. For example, if cleared for mild to moderate symptoms of stress, anxiety and depression, the self-assessment tool may be configured, for example, to help the user self-determine if they have the appropriate symptoms and whether those symptoms do, in fact, align with mild to moderate forms of the disease state. The DGC 600 may be configured so that such information may, for example, also form the basis or baseline for further self- and/or system-based evaluation

In some implementations, the initial self-evaluation tool 705 may help the player determine which games are best suited for the players motivational / play type (e.g., assessment engine 125), pathology (e.g., mental health concern) (e.g., patient pathology profile 260), and current state of dysfunction relative to the pathology (e.g., dynamic state profile 275)

The journaling module 710 may include an ongoing self-evaluation tool that, for example, may help the user 610 to determine which games are best suited for their health concern and current state of dysfunction. In some implementations, journaling may be accomplished as a micro journaling tool that may use AI and last situation / situational awareness to bring context to small, documented comments by the user 610. In some implementations, the journaling module may advantageously reduce the user's need to dedicate a lot of time to journaling or explanation of feelings. In some implementations, the journaling tool may include a method to allow the user 610 to rate their current state of being before, during or after play. These methods include but are not limited to, pick lists of words associated to games or current physical or mental health conditions the player is experiencing, recording spoken word with an option for translation to written words, using short or small sentences all of which are tied to the recent game playing experiences (situational awareness) that helps bring context to the information captured from the patient / player.

In this example, the DGC 600 further includes a game selection module 715. For example, the user 610 may use the game selection module 715 to select games based on their intended or associated use. In some implementations, the game selection module 715 may generate a suggested play list of games. For example, the list of games may be organized by indication for use, associated use, mechanism of action, genre, platform, and/or other similar users' recommendations. In some implementations, the game selection module 715 may generate a suggested next to play list of games. For example, the suggested next to play list of games may be generated based on a predetermined treatment path based on initial user inputs and/or standard treatment protocols and/or in response to users experience with previously played games or updates to the user self-evaluation tool or a diagnostic tool.

In some examples, the game selection module may suggest additional games based on the response and measured progress of the user 610. For example, the user 610 may make their own selection from a group of preselected games. In some implementations, the recommended games may be successful games recorded from the user 610's game history. In some examples, the therapeutic mechanisms that are used in the recommended games may be shown to the user 610. In some implementations, the game selection module 715 may recommend games based on past gaming history and/or statistic of their preference of games or preferences of other players with similar traits (including but not limited to health issues, similar reactions to treatment or other similar preferences for treatment) to advantageously drive more neural engagement to potentially achieve a higher rate of success. Further, users may be presented game choices from other platform users they follow or connected with so they may choose a similar gaming / treatment path.

The DGC 600, in this example, includes a cautions and warnings delivery module 720. For example, the cautions and warnings delivery module 720 may deliver cautions and warnings as required by the associated regulatory bodies. In some implementations, the DGC 600 may provide guidance to the user 610 prior to, during, and/or after a game to help focus the therapeutic intent of the DTx game 605. In some implementations, the warnings delivery module 720 may be implemented in the guidance and warning layer 405 described with reference to FIG. 4. In some implementations, the 720 may deliver pre- and/or post-amble explanations and/or suggestions for how to receive increased benefit from the play and/or therapy based on instructions for game play and/or use of the game as a motivational tool for therapeutic use.

The DGC 600 includes a platform communication module 725 to connect the DGC 600 to the game platform 615. In some implementations, the DGC 600 may be connected to the game platform 615 by a software and/or middleware-based connection. For example, the connection may allow the DGC 600 to know which game, for which duration the user 610 is playing.

The DGC 600 includes a game feedback module 730 and a feedback analysis module 735. In some implementations, the game feedback module 730 may recommend games for use based on their experience of using the games for therapeutic use. In some implementations, the feedback analysis module 735 may track the user 610 game play and resulting self-evaluation, including surveys, (micro) journaling and other forms of self-reflection and reporting to, for example, use the data gathered to improve suggested treatment paths for the specific DGC user. In some examples, the feedback analysis module 735 may use the data gathered to improve suggested treatment paths for other DGC users with similar needs, histories, and/or other cataloged traits.

The DGC 600, in some implementations, includes a medical professional module 740 (e.g., in the medical professional interface 215) to provide an interface for therapists and/or related medical professionals (e.g., Treatment Professionals) to view the DGC user's game play history, self-evaluations and other related reporting, including but not limited to journaling, microjournaling, user associations or any other information that may help a medical professional understand the current mental and / or physical health state of the user. For example, the medical professional module 740 may include means for Treatment Professionals to shape or suggest games, treatment mechanisms, themes, or directions for treatment or control other portions of the platform to improve the users treatment experience and outcomes. In some implementations, Treatment Professionals may use the medical professional module 740 to assign homework or other adjunctive work in conjunction with a patient's treatment plan, creating a coordinated treatment approach. The Treatment Professional may also use the information from the platform to create a more complete patient profile or understanding for the development of a more coordinated treatment plan.

In some implementations, the user may be provided with a control over data privacy for data logged and/or journaling data. The user may, for example, be provided with an option to transmit raw logging and/or journaling data to Treatment Professionals using the medical professional module 740. The user may, in some examples, be provided with an option to transmit summarized and/or redacted (e.g., manually, automatically) logging and/or journaling data to Treatment Professionals. In some implementations, by way of example and not limitation, the user may be provided a customized display of historical data (e.g., logging, journaling). For example, the DGC 600 may generate a different (e.g., customized) display of historical data to the Treatment Professional.

The DGC 600, in this example, includes a revenue module 745 to determine that the DGC was the source of recommendation for purchase and play of a game suitable of satisfying contractual obligations and calculations for a shared revenue agreement with a range of potential partners including but not limited to, game developers/publishers, Treatment Professionals, complementary platforms and other treatment or gaming related tools and services. For example, the revenue module 745 may determine that the DTx game 605 that the DGC 600 recommended before to the user 610 is played. For example, the revenue module may track usage data and charge access for the DGC 600. In some examples, the revenue module 745 may determine that a portion of the proceeds from purchasing the individual games may be chargeable.

The DGC 600 addresses the regulatory requirements using a regulatory module 750, in this example. In some implementations, the regulatory module 750 may advantageously minimize impact on game developers, and minizine ongoing verification and validation requirements of the game platform (e.g., centralizing around a web-based platform for intake and labeling and other required regulatory functions requiring ongoing verification and validation). For example, when the DGC 600 is delivering the DTx game 605, there may be regulatory requirements for all digital therapeutic games that make therapeutic claims that are not individually cleared as medical devices.

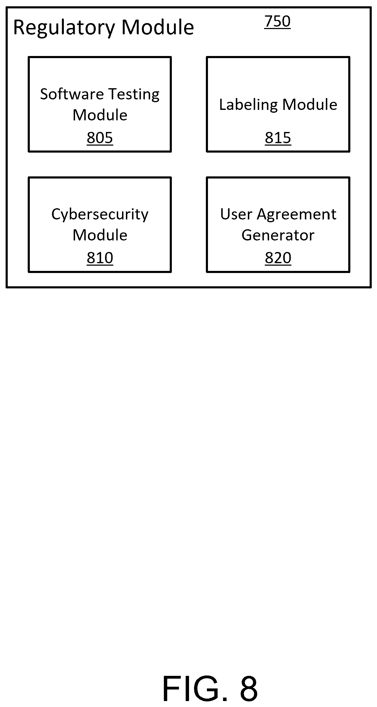

FIG. 8 depicts an exemplary regulatory module to address regulatory requirements for DTx games. For example, the DGC 600 may use the regulatory module 750 to meet at least some regulatory requirements for DTx games making therapeutic claims without being individually cleared as medical devices.

As shown, the regulatory module 750 includes a software testing module 805, a cybersecurity module 810, a labeling module 815, and a user agreement generator 820. For example, the software testing module 805 may perform software verification, validation, and hazard analysis to ensure that a device implements the therapy model as intended. The cybersecurity module 810 may, for example, provide appropriate cybersecurity protections compliant with authority (e.g, FDA premarket and post-market recommendations). For example, the labeling module 815 may include user instructions to instruct the patient to contact a physician before using the device. The user agreement generator 820, for example, may be prompted to acknowledge the recommendation to contact a physician before use. For example, the user agreement generator 820 may provide a standalone check-box separated from an end user license agreement. In some implementations, the end user license agreement may include a clear statement that the patient may contact a physician before using the device, even if the device is marketed directly to the consumer.

In some implementations, the end user license agreement may include user instructions to provide information about how to access additional resources related to the treatment of psychiatric conditions. In some examples, the instructions for use may include images that demonstrate how to interact with the device. For example, the conditions may include references to recommendations for the public made by healthcare professional organizations (e.g., the American Psychological Association or other associations, etc.). In some implementations, the end user license agreement may provide a clear description of the device's indication including the psychiatric condition/disorder the device is intended to treat and a description of the intended patient population (type of symptoms, duration, severity, and age range (adult vs child/adolescent) where it is expected to be effective, etc.). In some implementations, the labeling module 815 may provide a description of the therapeutic method (behavioral therapy, cognitive restructuring, etc.). In some implementations, the end user license agreement may provide a clear description of the recommended duration and frequency of use.

In some implementations, the end user license agreement may provide a summary of the clinical testing with the device. In various implementations, the summary may include an outline of available clinical performance testing including methods (e.g., clinical endpoints studied, study type, sample size, etc.) and results (e.g., group/responder analyses, performance goals, etc.). In some implementations, the summary may include a statement that the device has not been clinically tested and may therefore have unknown benefits and risks.

The end user license agreement may, for example, provide a description of the method of determining any treatment recommendations. In some examples, the end user license agreement may provide prominent notice to both the patient and health care provider, as applicable, that recommendations provided by the device are adjunctive (supporting) and may not be solely or primarily relied upon to treat psychiatric conditions. The end user license agreement may provide, in some implementations, a warning that the device does not represent a substitution for the patient's medication. In some examples, the end user license agreement may provide a statement as to whether the device is available with or without a prescription. In some examples, the end user license agreement may include instructions on when (under what circumstances, frequency) the user should consult a health care provider. The end user license agreement may also include, in some examples, a clear statement of what to do if the symptoms are not improving, and in what time period improvement should be expected. For example, the end user license agreement may include instructions on what to do in case of a medical emergency. The end user license agreement may include a clear identification of any device indications/functions that are not FDA-cleared, for example.

The DGC 600 may, in some implementations, deliver games as non-medical devices that encourage healthy lifestyles that are unrelated to specific diagnosis, cure, mitigation, prevention or treatment of a disease or condition. For example, the DGC 600 may include the General Wellness software functions unrelated to a specific disease or condition, such as, promoting relaxation, mindfulness, meditation, and/or reducing stress, fatigue or feelings of isolation due to the COVID-19 public health emergency or other circumstances that creates a isolation or perceived isolation for the user. For example, the DGC 600 may include the General Wellness software functions related to sleep, such as, for example, Promoting good sleep, Improving your sleep experience, Having more relaxing or restful sleep, Sleeping through the night or sleeping all night. For example, the DGC 600 may include the General Wellness software functions related to mental health or psychiatric conditions such as, for example, the DGC 600 provides motivational tips via text and other messages that are intended to reduce stress and promote a positive mental outlook, which may aid users in living well during the COVID-19 public health emergency or in cases of general or perceived isolation.

As an illustrative example, the DGC 600 may include a labeling engine (e.g., in the guidance and warning layer 405) configured to generate (digital) labeling to help individuals understand the cautions and warning associated with playing games with the expectation of relief from symptoms of mild / moderate Depression, Anxiety or Stress.

In various embodiments, upon a change being received at one of the DTx game 605, the therapeutic profile 280 of the changed media content is updated based on a predetermined set of validation rules, such that the changed media content is compliant with the validation rules and is fitted for a therapeutic intent.

FIG. 9 depicts an exemplary method 900 to validate wild games to become DTx games. For example, the method 900 may be performed by the regulatory module 750. In this example, the method 900 begins when an update of a validated DTx (e.g., the DTx module 155 in the data store 135) is received from the game platform in step 905. For example, the game platform 615 may inform the DGC 600 of an update using the interconnection between the game platform 615 and the DGC 600. For example, a game developer may change a part (e.g., a background color of one of the displays) of the unregulated game (e.g., the wild games 150).

In step 910, validation rules are retrieved. For example, the regulatory module 750 may retrieve the validation rules from the medical validation rules 220. Next, the updated DTx is evaluated based on the retrieved validation rules in step 915. For example, the software testing module 805 may test the updated component against the validation rules.

In a decision point 920, it is determined whether the updated DTx is compliant (e.g., to regulatory rules). If it is compliant, in step 925, an updated therapeutic profile of the DTx is generated, and the method 900 ends. For example, the therapeutic profile 280 may be updated based on the change in the DTx. For example, the labeling module 815 may generate appropriate labels based on the updated component to generate the updated DTx. Accordingly, the DTx may advantageously be verified to fit for a therapeutic intent.

If it is not compliant, in step 930, an error report is generated and the update is rejected, and the method 900 ends. For example, the error report may include the reasons for the rejection. Various embodiments of testing and validating a non-medical digital component of the medical digital asset are described in PCT Patent Application Serial No. PCT/US2021/071585, titled "Immersive Medicine Translational Engine for Development and Repurposing of Non-Verified and Validated Code," filed by Ryan J. Douglas, on September 24, 2021, the entire contents of which applications are incorporated herein by reference.

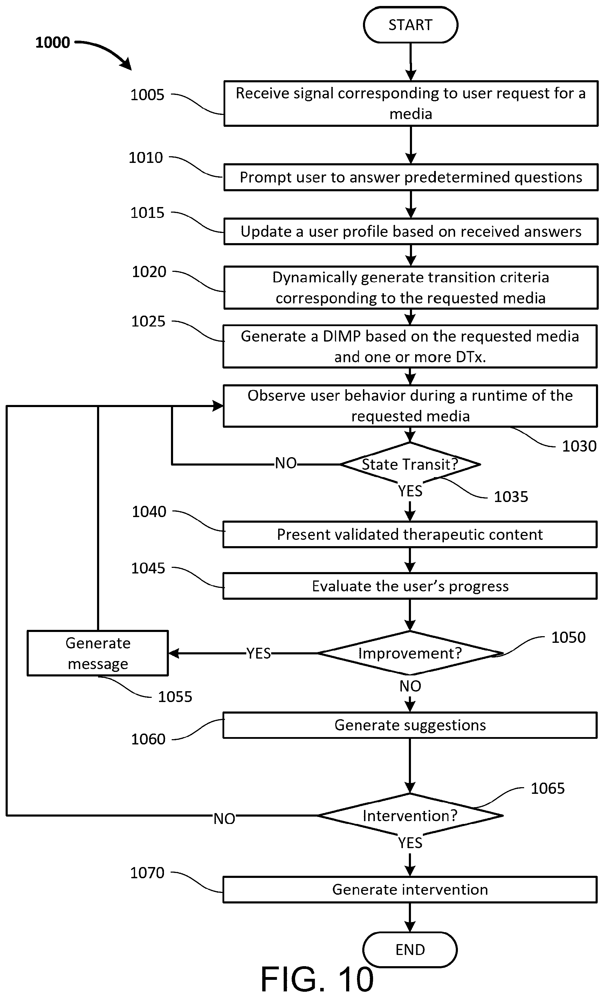

FIG. 10 depicts an exemplary method 1000 of delivering dynamic inducement media package (DIMP) and providing on-going monitoring to a user. For example, the MMPP 120 may be configured to perform the method 1000. The method 1000 begins when a signal corresponding to a user request for a media is received in step 1005. For example, the media requested may be an unregulated game (e.g., the wild games 150). In step 1010, the user is prompted to answer predetermined questions. For example, the assessment engine 125 may transmit questions to the device 106.

Next, in step 1015, a user profile is updated based on received answers. For example, the assessment engine 125 may update the user profile 140 based on received answers from the transmitted questions. After the user profile is updated, in step 1020, transition criteria corresponding to the requested media is dynamically generated. For example, the MMPGE 130 may generate the state transition criteria for a user-selected wild game. In step 1025, a DIMP is generated based on the requested media and one or more DTx.

After the DIMP is generated, in step 1030, user behavior is observed during a runtime of the requested media. For example, the observation layer 410 may observe the user behavior during a wild game 150 is being played. In a decision point 1035, it is determined whether state transit criteria are met. If the state transit criteria are not met, the step 1030 is repeated.

If the state transit criteria are met, validated therapeutic content is presented in step 1040. For example, the DIPSM 160 may transit the DIMP from the inducement state to the therapeutic state, and the MMPGE 130 may generate the therapeutic interface 170 to be presented at the device 106. In step 1045, the user's progress is evaluated. For example, the assessment engine 125 may evaluate a response received from the therapeutic interface 170.

In a decision point 1050, it is determined whether the user's health condition is improved. For example, the assessment engine 125 may evaluate specifically an anxiety of the user. If the user's health condition is improved, a notification message is generated in step 1055, and the step 1030 is repeated. If the user's health condition is not improved, suggestions are generated in step 1060. For example, the guidance and warning layer 405 may generate some suggestions based on the dynamic state profile 275 of the user.

In a decision point 1065, it is determined whether an intervention is needed. For example, the risk assessment layer 415 may determine, at the evaluation step, an intervention is needed when a condition of the user is below a predetermined intervention threshold. If intervention is not needed, the step 1030 is repeated.

If intervention is needed, in step 1070, an intervention is generated. For example, the risk assessment layer 415 may generate a message to the predetermined caretaker 270 to check the user.

Although various embodiments have been described with reference to the figures, other embodiments are possible. In some implementations, the DGC 600 (e.g., using the app as a 'wrapper' to deliver medical therapy associated with non-medical / non-regulated games) may, for example, advantageously enable distributors to deliver back catalog games with minimal intervention from the developer. It may, for example, provide new revenue streams to developers for existing games. It may, for example, enable game developers to associate games for good. It may, for example, enable delivery of powerful therapeutics to patients.

In some embodiments, the DGC 600 may monitor environmental conditions of gaming. In some embodiments, the DGC 600 may suggest games also available as standalone medical devices outside of the app environment.

As an illustrative example, a game (e.g., a "GameX") could be served up through a platform of the system. A diagnostic engine of the DGC 600 (e.g., part of the MMPGE 130, cooperating with the selection engine) may suggest a user has a particular issue. The system may suggest that GameX is associated with a highest historical success rate in addressing the particular issue (e.g., based on diagnostic factors, environmental factors, personality). GameX may be recommended. The DGC 600 may enable the user of GameX (e.g., GameX may not be medically approved and/or delivered) in association with medical therapy by providing labeling and/or therapeutic input and/or monitoring associated with playing GameX. The DGC 600 may monitor (e.g., through an API, through user input) how the interaction with GameX progressed. For example, the system may have feedback information from GameX. In some embodiments, by way of example and not limitation, information may include at least (a) did the user sign into the game (e.g., confirming the user played the game), and (b) duration of playing (e.g., when did the user start and stop playing the game).

In some embodiments, the MMDA 110 may not be a medical device. For example, the MMDA 110 may be configured to operate as a collection device. The MMDA 110 may cause the device 106 to transmit the data (e.g., unaltered) to the DGC 600 (e.g., a cloud server). Accordingly, the MMDA 110 or the device 106 may not need to be validated as a medical device. The medical device may, for example, be embodied in the cloud server(s) (e.g., the MMPP 120 or the DGC 600). The server-based system (e.g., including diagnostic engine, selection engine, monitoring engine) may be maintained, verified, and/or validated (e.g., according to regulatory controls). Accordingly, for example, the wild games 150 may be 'free to be games' and/or the app may be 'free to be an app on a phone' without requiring ongoing validations. As an illustrative example, a distributed network of servers may be managed for regulatory compliance (e.g., compliance with ISO 13485).

In some implementations, the MMDA 110 and/or the wild games 150 may be configured such that they cannot break the treatment mechanism. For example, the treatment mechanism may advantageously rely substantially entirely on the server-based system. The system may receive, analyze, and/or respond to information from a game and/or from the patients. The system may interact with the patients, for example, through the app. The system may send information to the game (e.g., to generate a display, to prompt for a report of playing time).

The cloud server(s) may, for example, be executing programs(s) of instruction(s) to recommend games that are played (e.g., via a diagnostic engine and/or selection engine), monitoring when the games are completed (e.g., via the monitoring engine), recommending that the patient perform therapeutically actions, and/or asking and checking what has been working for the patient. For example, the DGC 600 may provide interfaces (e.g., via the app, via a web interface) for the patient and/or a therapist. The DGC 600 may monitor who has been recommended what games and why.

In some embodiments, the DGC 600 may make a direct recommendation (e.g., "Hey, you look like the kind of person who should be playing GameX"). The DGC 600 may make a recommendation for an avenue to play GameX (e.g., "By the way, here's a really great place for you to go play GameX"). The DGC 600 may prompt for feedback (e.g., "Hey, you played GameX. How do you feel about it now?"). The DGC 600 may prompt for self-evaluation (e.g., "Hey, are you enjoying what you're doing? And how are you feeling about it?"). The response may, for example, be stored in structures associated with the games, users, and/or disorders. The game selection module 715 may update recommendations for the user and/or other users based on the feedback. Accordingly, the suggestions may, by way of example and not limitation, advantageously become more therapeutic and/or make user actions already being performed (e.g., playing a game they enjoy) more therapeutic.

In some implementations, the DGC 600 may, for example, generate hopeful suggestions (e.g., "Did you know that within three weeks of these feelings last time in a similar scenario, you indicated we were feeling quite a bit better?"). The DGC 600 may, for example, associate the hopeful suggestions with suggested actions (e.g., "By the way, this is what you did when you felt this way and you started feeling better after you did it"). The DGC 600 may make non-pressure suggestions (e.g., "You can start as soon as you ready and remember, they're games, it's fun! You had a good time. This is one of your favorites. Remember this game that you really liked? Why don't you try it for 20 minutes?")

In some implementations, the DIPGS 100 may not be associated with source code of the games. The DIPGS 100 may provide, for example, a code and/or link (e.g., via a visual indicia such as a logo, icon). Activating the code and/or link may direct the patient to a selected game curated by the DIPGS 100. The patient may, for example, already own the game and/or purchase it from a third party (e.g., a game platform).

In various embodiments, the games (e.g., the wild games 150, the DTx games 605) may be available on one or more platforms. For example, the games may be available through a third-party platform. The selection engine may indicate to the user one or more platforms available to access the game. The selection engine may generate an identifier associated with the user and/or with the recommendation. When the user accesses the game, a monitoring engine may associate the identifier with the user and/or the recommendation. The monitoring engine may determine, based on input received from an engine associated with the game (e.g., third-party game engine) and/or user input, attributes associated with the game play. Attributes may include, by way of example and not limitation, game start time, duration of play, game end time, or some combination thereof.

The monitoring engine may interact with the user before, during, and/or after gameplay. For example, the monitoring engine may provide cautions, warnings, and/or regulatory information to the user at the beginning of the game play. In some examples, the monitoring engine may provide guidance to the user regarding the game play (e.g., at the beginning of the game play). The monitoring engine may, for example, collect feedback from the user. For example, the monitoring engine may prompt the user for feedback about their experience, feelings, current status, improvements noticed, or some combination thereof. The monitoring engine may, for example, provide feedback to the selection engine. Accordingly, the selection engine may dynamically update recommendations and/or metadata associated with games based on the feedback received from the user and/or other.

The DGC 600 may suggest games to play at therapeutically relevant intervals. The DGC 600 may ask (e.g., prompt via the app) the player to think and/or do things while playing the game. The DGC 600 may check (e.g., via prompts) to monitor whether the user is following the suggestions. Accordingly, the DGC 600 may advantageously induce a change in the user's outlook. For example, the DGC 600 and process may result in a therapeutic treatment effect.

Such embodiments may, for example, advantageously integrate at one or more levels with a game to (selectively) apply a game to induce a positive treatment effect. For example, the system (e.g., through the app such as by user input, through an API associated with the system and the game) may receive feedback when the patient plays a game and for how long. The system may retrieve, for example, a list of games already integrated with the system. In some embodiments, one or more game(s) may be hosted and/or accessible via a platform associated with the system. At least some of the game(s) may, for example, be accessible by the user via the game's native environment (e.g., X-Box, Playstation, Wii). For example, the game may not be required to be hosted on a platform provided by the system to be therapeutically selected, delivered, and/or monitored. Such implementations may, for example, provide little to no integration burden for game developers. Accordingly, various such embodiments may advantageously provide expanded access to patients.

In various embodiments, the assessment engine 125 may retrieve, generate, and/or store information about a user's personality based on profiling (e.g., performed during a setup, performed periodically, provided by a therapist). The assessment engine 125 may, for example, steer the patient towards games that best fit their medical issues. The assessment engine 125 may, for example, record data relative to use such that, for example, the patient can 'self-steer' the app. In some embodiments, a third-party (e.g., practitioner therapist, doctor) may provide input to steer selection of games.

In various embodiments, information about suggestions, feedback, responses, engagement, duration of play, or some combination thereof, may be recorded in data structures (e.g., associated with the patient, associated with the disorder, associated with the games). The information may, for example, be analyzed for diagnostics and improvements in treatment (e.g., using an artificial intelligence model, such as a neural network).

Although an exemplary system has been described with reference to FIG. ***, other implementations may be deployed in other industrial, scientific, medical, commercial, and/or residential applications.

In some implementations, regulatory requirements may include a clear statement that the patient should contact a physician before using the device, even if the device is marketed directly to the consumer. The regulatory requirements may include labeling, including the user instructions, provide information about how to access additional resources related to the treatment of psychiatric conditions, such as references to recommendations for the public made by healthcare professional organizations, or such as the American Psychological Association or other associations. The regulatory requirements may include a clear description of the device's indication including the psychiatric condition/disorder the device is intended to treat and a description of the intended patient population (type of symptoms, duration, severity, and age range (adult vs child/adolescent) where it is expected to be effective, etc.).

For example, the regulatory requirements may include a description of the therapeutic method (e.g. cognitive behavioral psychotherapy etc.). For example, the regulatory requirements may include a clear description of the recommended duration and frequency of use. For example, the regulatory requirements may include instructions for use, including images that demonstrate how to interact with the device. For example, the regulatory requirements may include a summary of the clinical testing with the device. For example, the regulatory requirements may include description of the method of determining any treatment recommendations. For example, the regulatory requirements may include prominent notice to both the patient and health care provider, as applicable, that recommendations provided by the device are adjunctive (supporting) and should not be solely or primarily relied upon to treat psychiatric conditions. For example, the regulatory requirements may include a warning that the device does not represent a substitution for the patient's medication. For example, the regulatory requirements may include statements as to whether the device is available with or without a prescription. For example, the regulatory requirements may include instructions on when (under what circumstances, frequency) the user should consult a health care provider. For example, the regulatory requirements may include clear statements of what to do if the symptoms are not improving, and in what time period improvement should be expected. For example, the regulatory requirements may include instructions on what to do in case of a medical emergency. For example, the regulatory requirements may include clear identification of any device indications/functions that are not FDA-cleared.

In some implementations, by way of example and not limitation, various systems (e.g., DIMP generation, delivery, and/or monitoring system) may be configured to manage inducement exclusive of actual performance of therapy. For example, a DIPGS (e.g., such as disclosed herein) may manage delivery of inducement, monitoring of inducement, and/or monitoring surrounding inducement (e.g., to determine when to trigger inducement, to determine how to modify inducement, to determine when to terminate inducement). The DIPGS may, by way of example and not limitation, not actually deliver therapy. For example, a separate therapy delivery module may deliver the therapy. The DIPGS may, for example, generate an inducement package. The DIPGS may, for example, deliver an inducement. The DIPGS may, for example, release responsibility to a therapy module and/or human. The DIPGS may, for example, receive results of therapy. The DIPGS may, for example, generate updated inducement package(s) (e.g., DIMP(s)) based on results of therapy and/or inducement.

A MMPGE (e.g., such as depicted herein) may, for example, package an inducement and therapy. In some implementations, by way of example and not limitation, the MMPGE may, for example, operate exclusive of delivery of therapy. For example, the MMPGE may generate the packaged medical media and non-medical inducement (e.g., non-medical media), and provide the resulting media package for delivery. In some implementations, by way of example and not limitation, the MMPGE may, for example, monitor delivery. The MMPGE may, in some implementations, receive, from a separate module (e.g., a therapy module, a DIPGS ), results of therapy delivery. For example, in some implementations, a DIPGS and/or MMPGE may dynamically create and/or adjust a media package (e.g., a DIMP(s)) based on results of therapy and/or inducement, without delivering the therapy and/or without performing the therapy.

Computer program products may contain a set of instructions that, when executed by a processor device, cause the processor to perform prescribed functions. These functions may be performed in conjunction with controlled devices in operable communication with the processor. Computer program products, which may include software, may be stored in a data store tangibly embedded on a storage medium, such as an electronic, magnetic, or rotating storage device, and may be fixed or removable (e.g., hard disk, floppy disk, thumb drive, CD, DVD).

Although an example of a system, which may be portable, has been described with reference to the above figures, other implementations may be deployed in other processing applications, such as desktop and networked environments.

Temporary auxiliary energy inputs may be received, for example, from chargeable or single use batteries, which may enable use in portable or remote applications. Some embodiments may operate with other DC voltage sources, such as a 9V batteries, for example. Alternating current (AC) inputs, which may be provided, for example from a 50/60 Hz power port, or from a portable electric generator, may be received via a rectifier and appropriate scaling. Provision for AC (e.g., sine wave, square wave, triangular wave) inputs may include a line frequency transformer to provide voltage step-up, voltage step-down, and/or isolation.

Although particular features of an architecture have been described, other features may be incorporated to improve performance. For example, caching (e.g., L1, L2, ...) techniques may be used. Random access memory may be included, for example, to provide scratch pad memory and or to load executable code or parameter information stored for use during runtime operations. Other hardware and software may be provided to perform operations, such as network or other communications using one or more protocols, wireless (e.g., infrared) communications, stored operational energy and power supplies (e.g., batteries), switching and/or linear power supply circuits, software maintenance (e.g., self-test, upgrades), and the like. One or more communication interfaces may be provided in support of data storage and related operations.

Some systems may be implemented as a computer system that can be used with various implementations. For example, various implementations may include digital circuitry, analog circuitry, computer hardware, firmware, software, or combinations thereof. Apparatus can be implemented in a computer program product tangibly embodied in an information carrier, e.g., in a machine-readable storage device, for execution by a programmable processor; and methods can be performed by a programmable processor executing a program of instructions to perform functions of various embodiments by operating on input data and generating an output. Various embodiments can be implemented advantageously in one or more computer programs that are executable on a programmable system including at least one programmable processor coupled to receive data and instructions from, and to transmit data and instructions to, a data storage system, at least one input device, and/or at least one output device. A computer program is a set of instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

Suitable processors for the execution of a program of instructions include, by way of example, both general and special purpose microprocessors, which may include a single processor or one of multiple processors of any kind of computer. Generally, a processor will receive instructions and data from a read-only memory or a random-access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including, by way of example, semiconductor memory devices, such as EPROM, EEPROM, and flash memory devices; magnetic disks, such as internal hard disks and removable disks; magneto-optical disks; and CD-ROM and DVD-ROM disks. The processor and the memory can be supplemented by, or incorporated in, ASICs (application-specific integrated circuits).

In some implementations, each system may be programmed with the same or similar information and/or initialized with substantially identical information stored in volatile and/or non-volatile memory. For example, one data interface may be configured to perform auto configuration, auto download, and/or auto update functions when coupled to an appropriate host device, such as a desktop computer or a server.

In some implementations, one or more user-interface features may be custom configured to perform specific functions. Various embodiments may be implemented in a computer system that includes a graphical user interface and/or an Internet browser. To provide for interaction with a user, some implementations may be implemented on a computer having a display device. The display device may, for example, include an LED (light-emitting diode) display. In some implementations, a display device may, for example, include a CRT (cathode ray tube). In some implementations, a display device may include, for example, an LCD (liquid crystal display). A display device (e.g., monitor) may, for example, be used for displaying information to the user. Some implementations may, for example, include a keyboard and/or pointing device (e.g., mouse, trackpad, trackball, joystick), such as by which the user can provide input to the computer.

In various implementations, the system may communicate using suitable communication methods, equipment, and techniques. For example, the system may communicate with compatible devices (e.g., devices capable of transferring data to and/or from the system) using point-to-point communication in which a message is transported directly from the source to the receiver over a dedicated physical link (e.g., fiber optic link, point-to-point wiring, daisy-chain). The components of the system may exchange information by any form or medium of analog or digital data communication, including packet-based messages on a communication network. Examples of communication networks include, e.g., a LAN (local area network), a WAN (wide area network), MAN (metropolitan area network), wireless and/or optical networks, the computers and networks forming the Internet, or some combination thereof. Other implementations may transport messages by broadcasting to all or substantially all devices that are coupled together by a communication network, for example, by using omni-directional radio frequency (RF) signals. Still other implementations may transport messages characterized by high directivity, such as RF signals transmitted using directional (i.e., narrow beam) antennas or infrared signals that may optionally be used with focusing optics. Still other implementations are possible using appropriate interfaces and protocols such as, by way of example and not intended to be limiting, USB 2.0, Firewire, ATA/IDE, RS-232, RS-422, RS-485, 802.11 a/b/g, Wi-Fi, Ethernet, IrDA, FDDI (fiber distributed data interface), token-ring networks, multiplexing techniques based on frequency, time, or code division, or some combination thereof. Some implementations may optionally incorporate features such as error checking and correction (ECC) for data integrity, or security measures, such as encryption (e.g., WEP) and password protection.

In various embodiments, the computer system may include Internet of Things (IoT) devices. IoT devices may include objects embedded with electronics, software, sensors, actuators, and network connectivity which enable these objects to collect and exchange data. IoT devices may be in-use with wired or wireless devices by sending data through an interface to another device. IoT devices may collect useful data and then autonomously flow the data between other devices.

Various examples of modules may be implemented using circuitry, including various electronic hardware. By way of example and not limitation, the hardware may include transistors, resistors, capacitors, switches, integrated circuits, other modules, or some combination thereof. In various examples, the modules may include analog logic, digital logic, discrete components, traces and/or memory circuits fabricated on a silicon substrate including various integrated circuits (e.g., FPGAs, ASICs), or some combination thereof. In some embodiments, the module(s) may involve execution of preprogrammed instructions, software executed by a processor, or some combination thereof. For example, various modules may involve both hardware and software.

In an illustrative aspect, a dynamic inducement package generation system (DIPGS) (e.g., 100) may, for example, include a data store. The data store may, for example, include validated digital therapeutics (e.g., 155), therapeutic profiles (e.g., 280) each associated with one of the validated therapeutics, a user profile (e.g., 140) associated with a user, and media profiles (e.g., 145) associated with wild videogames (e.g., 150).

The DIPGS may, for example, include an assessment engine (e.g., 125) coupled to the data store, and configured to generate the user profile based on a journal (e.g., 255) of user input.

The DIPGS may, for example, include a medical media package generation engine (e.g., 130) coupled to the data store, wherein the medical media package generation engine may, for example, be configured to generate a dynamic inducement media package (DIMP). The DIMP may, for example, include one or more of the validated digital therapeutics based on the user profile, the therapeutic profiles, and a media profile associated with a user-selected wild videogame. The DIMP may, for example, include state transition criteria generated based on the user profile and the validated digital therapeutics in the DIMP, wherein, when the state transition criteria are met, an operating state of the DIMP may, for example, transition from an inducement mode to a therapeutic mode.

The DIMP may, for example, be configured to be run in a user device (e.g., 106) remotely coupled to the dynamic inducement package generation system. In a runtime of the DIMP, in the inducement mode, the user-selected wild videogame may, for example, be played regularly. When runtime conditions have met the state transition criteria, the DIMP may, for example, transition from the inducement mode to a therapeutic mode, and the medical media package generation engine may, for example, generate a therapeutic immersed media package (TIMP). The TIMP may, for example, include a recommended set of validated media content based on a therapeutic profile of the validated digital therapeutics and the user profile. The recommended set of validated media content may, for example, be presented at a therapeutic interface at the user device.

In the therapeutic mode, the medical media package generation engine may, for example, generate an interventive monitoring content (IMC). The IMC may, for example, include the TIMP such that the DIMP switches from running the user-selected wild videogame to the validated digital therapeutics. Upon receiving a response of the IMC, the assessment engine may, for example, update the user profile based on the received response, such that medical guidance and progress monitor are continuously and automatically provided to the user.

The validated media content may, for example, includes a set of questions.

The DIPGS further may, for example, include a dynamic inducement package state machine (e.g., DIPSM) configured to, during a runtime of the DIMP, (a) identify at least one transition point in the DIMP, (b) transition the runtime of the DIMP from the inducement mode to the therapeutic mode, (c) present the TIMP at the user device, and, (d) transmit a signal to the assessment engine to update the user profile based on a result of the TIMP.

The DIPSM may, for example, be further configured to adjust a set of predetermined runtime parameters of the DIMP based on the TIMP and the therapeutic profile of the validated media content in the TIMP and the user profile.

The switch from running the user-selected wild videogame to the validated digital therapeutics may, for example, include inserting an instruction in the wild videogame.

The switch from running the user-selected wild videogame to the validated digital therapeutics may, for example, include interrupting the runtime of the DIMP during playing of the wild videogame.

The DIPGS further may, for example, include an observation layer, wherein the observation layer may, for example, be configured to observe the user's behavior to collection health metric information, and, based on the collected information, solicit responses from the user.

When presentation of the DIMP may, for example, be not suitable, the medical media package generation engine may, for example, be configured to generate a streamlined DIMP. The DIMP may, for example, include the TIMP based on the user profile and a history of played DIMPs.

The TIMP may, for example, includes guidance and warnings to the user. The guidance and warnings may, for example, be determined to be presented based on the state transition criteria. The guidance and warnings may, for example, include instructions to induce autonomic function using voluntary action required in playing the DIMP.

The DIPGS further may, for example, include a regulatory module. The regulatory module may, for example, be configured to, upon a change to one of the validated digital therapeutics being received, update a therapeutic profile of the changed digital therapeutics based on a predetermined set of validation rules such that the changed digital therapeutics may, for example, be verified to fit for a therapeutic intent.
In an illustrative aspect, a dynamic inducement package generation system (DIPGS) (e.g., 100) may, for example, include. a data store. The data store may, for example, include validated digital therapeutics (e.g., 155), a user profile (e.g., 140) associated with a user, and media profiles (e.g., 145) associated with wild media content. The DIPGS may, for example, include an assessment engine (e.g., 125) coupled to the data store, and configured to generate the user profile based on a journal (e.g., 255) of user input. The DIPGS may, for example, include a medical media package generation engine (e.g., 130) coupled to the data store, wherein the medical media package generation engine may, for example, be configured to generate a dynamic inducement media package (e.g., DIMP) based on the user profile, and a media profile associated with a user-selected wild media content.

The DIMP may, for example, include state transition criteria, and the DIMP may, for example, be configured to be run in a user device remotely coupled to the dynamic inducement package generation system, such that, in a runtime of the DIMP, the DIMP operates in an in an inducement mode and/or in a therapeutic mode.

In an inducement mode, the user-selected wild media content may, for example, be played regularly. When runtime conditions have met the state transition criteria, the DIMP may, for example, transition from the inducement mode to a therapeutic mode. In the therapeutic mode, the medical media package generation engine generates an interventive monitoring content (e.g., IMC). The IMC may, for example, include at least one of the multiple validated digital therapeutics such that the DIMP switches from running the user-selected wild media content to the validated digital therapeutics. Upon receiving a response of the IMC, the assessment engine may, for example, update the user profile based on the received response, such that medical guidance and progress monitor are continuously and automatically provided to the user.
The medical media package generation engine may, for example, configured to dynamically generate the state transition criteria based on the user profile and the validated digital therapeutics in the DIMP, identify at least one transition point in the DIMP, wherein, at each of the transition point, the runtime of the DIMP transits from the inducement mode to the therapeutic mode, and, generate a therapeutic immersed media package (e.g., TIMP). The TIMP may, for example, include a recommended set of validated media content at each of the identified transition points based on a therapeutic profile of the validated digital therapeutics and the user profile. The recommended set of validated media content may, for example, be configured to be presented (e.g., automatically) at a therapeutic interface at the user device.

The validated media content may, for example, include a set of questions.

The DIPGS further may, for example, include a dynamic inducement package state machine (e.g., DIPSM) configured to, during a runtime of the DIMP, (a) identify at least one transition point in the DIMP, (b) transition the runtime of the DIMP from the inducement mode to the therapeutic mode, (c) present the TIMP at the user device, and, (d) transmit a signal to the assessment engine to update the user profile based on a result of the TIMP.

The DIPSM may, for example, further be configured to adjust a set of predetermined runtime parameters of the DIMP based on the TIMP and the therapeutic profile of the validated media content in the TIMP and the user profile.

The switch from running the user-selected wild media content to the validated digital therapeutics may, for example, include inserting an instruction in the wild media content.

The switch from running the user-selected wild media content to the validated digital therapeutic(s) may, for example, include interrupting the runtime of the DIMP during playing of the wild media content.

The DIPGS further may, for example, include an observation layer, wherein the observation layer may, for example, configured to observe the user's behavior to collection health metric information, and, based on the collected information, solicit responses from the user.

The DIPGS may be configured such that, when presentation of the DIMP may, for example, be not suitable (e.g., based on one or more predetermined suitability criterions, based on user discretion and/or input), the medical media package generation engine may, for example, be configured to generate a streamlined DIMP. The streamlined DIMP may, for example, include the TIMP based on the user profile and a history of played DIMPs.

The TIMP may, for example, include guidance and warnings to the user, wherein the guidance and warnings are determined to be presented based on the state transition criteria. The guidance and warnings may, for example, include instructions to induce autonomic function using voluntary action required in playing the DIMP.

The DIPGS further may, for example, include a regulatory module, wherein, the regulatory module may, for example, be configured to, upon receiving a change to one of the validated digital therapeutics, update a therapeutic profile of the changed digital therapeutics based on a predetermined set of validation rules such that the changed digital therapeutics may, for example, be verified to fit for a therapeutic intent.

The dynamic inducement package generation system of claim 12, wherein the wild media content may, for example, include a videogame.

In an illustrative aspect, a computer-implemented method may be performed by at least one processor to automatically provide dynamically generated immersive treatment mechanisms based monitoring and tracking mechanism in an unregulated media content environment. The method may, for example, include receive a signal corresponding to a user request for playing a wild media content (e.g., 1005). The method may, for example, include dynamically generate a set of transition criteria based on a user profile and a therapeutic profile of the requested media content (e.g., 1020). The method may, for example, include observe a user behavior during a runtime of the media content (e.g., 1030). The method may, for example, include, when the set of transition criteria is met, transit the runtime of the media content from an inducement mode to a therapeutic mode (e.g., 1035). The method may, for example, include, in the therapeutic mode, dynamically generate a validated therapeutic content including questions and guidance generated based on the user profile (e.g., 1040). The method may, for example, include, based on response received from the validated therapeutic content and observed user behavior, evaluate the user's progress (e.g., 1045). The method may, for example, include generate intervention if the user's progress is below an intervention threshold (e.g., 1065), such that medical guidance and progress monitor of therapeutic process are continuously provided.

In an illustrative aspect, a computer program product (e.g., CPP) may, for example, include a program of instructions tangibly embodied on a non-transitory computer readable medium wherein, when the instructions are executed on a processor, the processor causes dynamic inducement media package generation and presentation operations to be performed to automatically provide dynamically generated immersive treatment mechanisms based monitoring and tracking mechanism in an unregulated media content environment. The operations may, for example, include receive a signal corresponding to a user request for playing a wild media content (e.g., 1005). The operations may, for example, include dynamically generate a set of transition criteria based on a user profile and a therapeutic profile of the requested media content (e.g., 1020). The operations may, for example, include observe a user behavior during a runtime of the media content (e.g., 1030). The operations may, for example, include, when the set of transition criteria is met, transit the runtime of the media content from an inducement mode to a therapeutic mode (e.g., 1035). The operations may, for example, include, in the therapeutic mode, dynamically generate a validated therapeutic content including questions and guidance generated based on the user profile (e.g., 1040). The operations may, for example, include, based on response received from the validated therapeutic content and observed user behavior, evaluate the user's progress (e.g., 1045). The operations may, for example, include, generate intervention if the user's progress may, for example, be below a intervention threshold (e.g., 1065), such that medical guidance and progress monitor of therapeutic process are continuously provided.

A number of implementations have been described. Nevertheless, it will be understood that various modifications may be made. For example, advantageous results may be achieved if the steps of the disclosed techniques were performed in a different sequence, or if components of the disclosed systems were combined in a different manner, or if the components were supplemented with other components.

## Claims

1. A dynamic inducement package generation system (100) configured to dynamically generate immersive treatment mechanisms capable of use for self-treatment comprising:
a data store (135) comprising:
a plurality of validated digital therapeutics (155),
a plurality of therapeutic profiles (280) each associated with one of the plurality of validated digital therapeutics,
a journal (255) of user input associated with the user, the journal comprising: a history of user responses to journaling prompts, and a history of interactions of the user with media in an inducement state,
a user profile (140) associated with a user and comprising temporally distributed history of the user and attributes of the user including pathology attributes and motivation attributes, and
a plurality of media profiles (145) associated with a plurality of publicly accessible wild videogames at least for non-medical use (150), wherein wild videogames include one or more of games, music, or videos, the plurality of media profiles corresponding to specific media objects of the plurality of wild videogames and including predetermined media attributes and digital therapy transition points;
an assessment engine (125) coupled to the data store, wherein the assessment engine is configured to generate the user profile associated with the user based on the journal of user input retrieved from the data store, wherein generating the user profile comprises generating an initial user profile, and update the user profile at least based on the user responses to journaling prompts; and,
a medical media package generation engine (130) coupled to the data store, wherein the medical media package generation engine is configured to generate a dynamic inducement media package (DIMP) comprising:
one or more of the plurality of validated digital therapeutics selected based on: the user profile, including the temporally distributed history, the pathology attributes and the motivation attributes; the plurality of therapeutic profiles; and a media profile, of the plurality of media profiles, associated with a user-selected wild videogame of the plurality of wild videogames,
state transition criteria generated based on the user profile and the validated digital therapeutics in the DIMP, wherein:
the state transition criteria include at least one of a time criterion and a stop playing criterion for permitting a transition between an inducement mode and a therapeutic mode, and
when the state transition criteria are met, an operating state of the DIMP transitions from the inducement mode to the therapeutic mode,
wherein:
the DIMP is configured to be processed in a user device (106) remotely coupled to the dynamic inducement package generation system, and wherein, in a runtime of the DIMP,
in the inducement mode, the user-selected wild videogame is played,
when runtime conditions have met the state transition criteria, the DIMP transits from the inducement mode to a therapeutic mode, and the medical media package generation engine generates a therapeutic immersed media package (TIMP) comprising a recommended set of validated media content selected based on the therapeutic profiles of the validated digital therapeutics and based on the user profile, wherein the recommended set of validated media content is presented at a therapeutic interface at the user device,
in the therapeutic mode, the medical media package generation engine generates an interventive monitoring content (IMC) comprising the TIMP such that the DIMP switches from running the user-selected wild videogame to the validated digital therapeutics, and,
upon receiving a response of the IMC, the assessment engine updates the user profile based on the received response, such that a subsequent DIMP is generated based on the received response.

2. The dynamic inducement package generation system of claim 1, wherein the validated media content comprises a set of questions.

3. The dynamic inducement package generation system of claim 1, further comprising a dynamic inducement package state machine (DIPSM) configured to, during a runtime of the DIMP,
(a) identifies at least one transition point in the DIMP,
(b) transit the runtime of the DIMP from the inducement mode to the therapeutic mode,
(c) presents the TIMP at the user device, and,
(d) transmit a signal to the assessment engine to update the user profile based on a result of the TIMP.

4. The dynamic inducement package generation system of claim 3, wherein the DIPSM is further configured to adjust a set of predetermined runtime parameters of the DIMP based on the TIMP and the therapeutic profile of the validated media content in the TIMP and the user profile.

5. The dynamic inducement package generation system of claim 1, wherein the switch from running the user-selected wild videogame to the validated digital therapeutics comprises inserting an instruction in the wild videogame.

6. The dynamic inducement package generation system of claim 1, wherein the switch from running the user-selected wild videogame to the validated digital therapeutics comprises interrupting the runtime of the DIMP during playing of the wild videogame.

7. The dynamic inducement package generation system of claim 1, further comprising an observation layer, wherein the observation layer is configured to
observe the user's behavior to collect health metric information, and,
based on the collected information, solicit responses from the user.

8. The dynamic inducement package generation system of claim 1, wherein:
the medical media package generation engine is configured to, when presentation of the DIMP is not suitable based on environment limitations comprising a non-game appropriate environment, generate a streamlined DIMP comprising the TIMP based on the user profile and a history of played DIMPs.

9. The dynamic inducement package generation system of claim 2, wherein the TIMP comprises guidance and warnings to the user, wherein the guidance and warnings are determined to be presented based on the state transition criteria.

10. The dynamic inducement package generation system of claim 9, wherein the guidance and warnings comprise instructions to induce autonomic function of the user's body using voluntary action required in playing the DIMP.

11. The dynamic inducement package generation system of claim 1, further comprising a regulatory module, wherein:
the regulatory module is configured to, upon receiving a change to one of the validated digital therapeutics, update a therapeutic profile of the changed digital therapeutics based on a predetermined set of validation rules, wherein:
if the changed digital therapeutics is compliant to the predetermined set of validation rules, the updated therapeutic profile is generated, and,
otherwise, the change to one of the validated digital therapeutics is rejected, such that the changed digital therapeutics is verified to fit for a therapeutic intent.

12. A computer program product (CPP) comprising a program of instructions tangibly embodied on a non-transitory computer readable medium wherein, when the instructions are executed on a processor, cause the processor to perform operations of a dynamic inducement package generation system (100) for immersive treatment mechanisms, the system being configured to:
provide access to a data store (135) comprising:
a plurality of validated digital therapeutics (155),
a plurality of therapeutic profiles (280) each associated with one of the plurality of validated digital therapeutics,
a journal (255) of user input associated with the user, the journal comprising: a history of user responses to journaling prompts, and a history of interactions of the user with media in an inducement state,
a user profile (140) associated with a user and comprising temporally distributed history of the user and attributes of the user including pathology attributes and motivation attributes, and
a plurality of media profiles (145) associated with a plurality of publicly accessible wild games at least for non-medical use (150), wherein wild games including one or more of games, music, or videos, the plurality of media profiles corresponding to specific media objects of the plurality of wild games and including predetermined media attributes and digital therapy transition points;
operate an assessment engine (125) coupled to the data store, wherein the assessment engine is configured to generate the user profile associated with the user based on the journal of user input retrieved from the data store, wherein generating the user profile comprises generating an initial user profile, and update the user profile at least based on the user responses to journaling prompts; and,
operate a medical media package generation engine (130) coupled to the data store, wherein the medical media package generation engine is configured to generate a dynamic inducement media package (DIMP) comprising:
one or more of the plurality of validated digital therapeutics selected based on: the user profile, including the temporally distributed history, the pathology attributes and the motivation attributes; the plurality of therapeutic profiles; and a media profile, of the plurality of media profiles, associated with a user-selected wild game of the plurality of wild games,
state transition criteria generated based on the user profile and the validated digital therapeutics in the DIMP, wherein:
the state transition criteria include at least one of a time criterion and a stop playing criterion for permitting a transition between an inducement mode and a therapeutic mode, and
when the state transition criteria are met, an operating state of the DIMP transitions from the inducement mode to the therapeutic mode,
facilitate operations of the DIMP on a user device (106) remotely coupled to the dynamic inducement package generation system (100), wherein, in a runtime of the DIMP:
in the inducement mode, the user-selected wild game is played,
when runtime conditions have met the state transition criteria, the DIMP transits from the inducement mode to a therapeutic mode, and the medical media package generation engine generates a therapeutic immersed media package (TIMP) comprising a recommended set of validated media content selected based on the therapeutic profiles of the validated digital therapeutics and based on the user profile, wherein the recommended set of validated media content is presented at a therapeutic interface at the user device,
in the therapeutic mode, the medical media package generation engine generates an interventive monitoring content (IMC) comprising the TIMP such that the DIMP switches from running the user-selected wild game to the validated digital therapeutics, and,
upon receiving a response of the IMC, the assessment engine updates the user profile based on the received response, such that a subsequent DIMP is generated based on the received response.

13. The computer program product of claim 12, wherein the validated media content comprises a set of questions.

14. The computer program product of claim 12, wherein the instructions further cause the processor to operate a dynamic inducement package state machine (DIPSM) configured to, during a runtime of the DIMP,
(a) identify at least one transition point in the DIMP,
(b) transit the runtime of the DIMP from the inducement mode to the therapeutic mode,
(c) present the TIMP at the user device, and,
(d) transmit a signal to the assessment engine to update the user profile based on a result of the TIMP.

15. The dynamic inducement package generation system of claim 14, wherein the DIPSM is further configured to adjust a set of predetermined runtime parameters of the DIMP based on the TIMP and the therapeutic profile of the validated media content in the TIMP and the user profile.

## Patentansprüche

1. Dynamisches Anreizpaket-Erzeugungssystem (100), das ausgebildet ist, dynamisch immersive Behandlungsmechanismen zu erzeugen, die zur Selbstbehandlung verwendet werden können, aufweisend:
einen Datenspeicher (135), aufweisend:
eine Mehrzahl validierter digitaler Therapeutika (155),
eine Mehrzahl von therapeutischen Profilen (280), die jeweils einem von der Mehrzahl validierter digitaler Therapeutika zugeordnet sind,
ein Protokoll (255) mit Benutzereingaben, die dem Benutzer zugeordnet sind, wobei das Protokoll aufweist: eine Historie von Benutzerantworten auf Protokollierungsaufforderungen und eine Historie von Interaktionen des Benutzers mit Medien in einem Anreizzustand,
ein Benutzerprofil (140), das einem Benutzer zugeordnet ist und eine zeitlich verteilte Historie des Benutzers und Attribute des Benutzers aufweist, einschließlich Pathologieattributen und Motivationsattributen, und
eine Mehrzahl von Medienprofilen (145), die einer Mehrzahl von öffentlich zugänglichen Wild-Videospielen zumindest für nicht-medizinische Zwecke (150) zugeordnet sind, wobei Wild-Videospiele eines oder mehrere der folgenden Elemente beinhalten: Spiele, Musik oder Videos, wobei die Mehrzahl von Medienprofilen bestimmten Medienobjekten von der Mehrzahl von Wild-Videospielen entspricht und vorbestimmte Medienattribute und digitale Therapieübergangspunkte beinhaltet;
eine mit dem Datenspeicher gekoppelte Bewertungs-Engine (125), wobei die Bewertungs-Engine ausgebildet ist, das dem Benutzer zugeordnete Benutzerprofil auf der Grundlage des aus dem Datenspeicher abgerufenen Protokolls mit Benutzereingaben zu erzeugen, wobei das Erzeugen des Benutzerprofils ein Erzeugen eines anfänglichen Benutzerprofils und ein Aktualisieren des Benutzerprofils zumindest auf der Grundlage der Benutzerantworten auf Protokollierungsaufforderungen aufweist; und
eine mit dem Datenspeicher gekoppelte Engine zur Erzeugung medizinischer Medienpakete (130), wobei die Engine zur Erzeugung medizinischer Medienpakete ausgebildet ist, ein dynamisches Anreizmedienpaket (DIMP) zu erzeugen, aufweisend:
ein oder mehrere von der Mehrzahl validierter digitaler Therapeutika, die ausgewählt werden auf der Grundlage: des Benutzerprofils, einschließlich der zeitlich verteilten Historie, der Pathologieattribute und der Motivationsattribute; der Mehrzahl von therapeutischen Profilen; und eines Medienprofils aus der Mehrzahl von Medienprofilen, das einem von dem Benutzer ausgewählten Wild-Videospiel aus der Mehrzahl von Wild-Videospielen zugeordnet ist,
Zustandsübergangskriterien, die auf der Grundlage des Benutzerprofils und der validierten digitalen Therapeutika in dem DIMP erzeugt werden, wobei:
die Zustandsübergangskriterien mindestens eines von einem Zeitkriterium und einem Spielstoppkriterium beinhalten, um einen Übergang zwischen einem Anreizmodus und einem therapeutischen Modus zu ermöglichen, und
wenn die Zustandsübergangskriterien erfüllt sind, ein Betriebszustand des DIMP von dem Anreizmodus in den therapeutischen Modus übergeht,
wobei:
das DIMP ausgebildet ist, in einem Benutzergerät (106) verarbeitet zu werden, das mit dem dynamischen Anreizpaket-Erzeugungssystem entfernt gekoppelt ist, und wobei in einer Laufzeit des DIMP
in dem Anreizmodus das von dem Benutzer ausgewählte Wild-Videospiel gespielt wird,
wenn Laufzeitbedingungen die Zustandsübergangskriterien erfüllt haben, das DIMP von dem Anreizmodus in einen therapeutischen Modus wechselt, und die Engine zur Erzeugung medizinischer Medienpakete ein therapeutisches immersives Medienpaket (TIMP) erzeugt, das einen empfohlenen Satz validierter Medieninhalte aufweist, die auf der Grundlage der therapeutischen Profile der validierten digitalen Therapeutika und auf der Grundlage des Benutzerprofils ausgewählt wurden, wobei der empfohlene Satz validierter Medieninhalte auf einer therapeutischen Schnittstelle auf dem Benutzergerät präsentiert wird,
in dem therapeutischen Modus die Engine zur Erzeugung medizinischer Medienpakete einen interventionellen Überwachungsinhalt (IMC), der das TIMP aufweist, erzeugt, sodass das DIMP von der Ausführung des von dem Benutzer ausgewählten Videospiels zu den validierten digitalen Therapeutika umschaltet, und
nach Empfang einer Antwort des IMC die Bewertungs-Engine das Benutzerprofil auf der Grundlage der empfangenen Antwort aktualisiert, so dass ein nachfolgendes DIMP auf der Grundlage der empfangenen Antwort erzeugt wird.

2. Dynamisches Anreizpaket-Erzeugungssystem nach Anspruch 1, wobei der validierte Medieninhalt einen Satz von Fragen aufweist.

3. Dynamisches Anreizpaket-Erzeugungssystem nach Anspruch 1, zudem aufweisend eine dynamische Anreizpaket-Zustandsmaschine (DIPSM), die so ausgebildet ist, dass sie während einer Laufzeit des DIMP
(a) mindestens einen Übergangspunkt in dem DIMP identifiziert,
(b) die Laufzeit des DIMP von dem Anreizmodus in den therapeutischen Modus überführt,
(c) das TIMP auf dem Benutzergerät darstellt, und
(d) ein Signal an die Bewertungs-Engine sendet, um das Benutzerprofil auf der Grundlage eines Ergebnisses des TIMP zu aktualisieren.

4. Dynamisches Anreizpaket-Erzeugungssystem nach Anspruch 3, wobei die DIPSM zudem ausgebildet ist, einen Satz vorbestimmter Laufzeitparameter des DIMP auf der Grundlage des TIMP und des therapeutischen Profils des validierten Medieninhalts in dem TIMP und dem Benutzerprofil anzupassen.

5. Dynamisches Anreizpaket-Erzeugungssystem nach Anspruch 1, wobei das Umschalten von der Ausführung des von dem Benutzer ausgewählten Wild-Videospiels zu den validierten digitalen Therapeutika ein Einfügen einer Anweisung in das Wild-Videospiel aufweist.

6. Dynamisches Anreizpaket-Erzeugungssystem nach Anspruch 1, wobei das Umschalten von der Ausführung des von dem Benutzer ausgewählten Wild-Videospiels zu den validierten digitalen Therapeutika ein Unterbrechen der Laufzeit des DIMP während des Spielens des Wild-Videospiels aufweist.

7. Dynamisches Anreizpaket-Erzeugungssystem nach Anspruch 1, zudem aufweisend eine Beobachtungsschicht, wobei die Beobachtungsschicht ausgebildet ist,
das Verhalten des Benutzers zu beobachten, um Gesundheitsdaten-Informationen zu sammeln, und
auf der Grundlage der gesammelten Informationen Antworten von dem Benutzer anzufordern.

8. Dynamisches Anreizpaket-Erzeugungssystem nach Anspruch 1, wobei:
die Engine zur Erzeugung medizinischer Medienpaketen ausgebildet ist, dass sie, wenn eine Präsentation des DIMP aufgrund von Umgebungsbeschränkungen, die eine für Spiele ungeeignete Umgebung aufweisen, ungeeignet ist, ein optimiertes DIMP erzeugt, das das TIMP auf der Grundlage des Benutzerprofils und einer Historie von gespielten DIMPs aufweist.

9. Dynamisches Anreizpaket-Erzeugungssystem nach Anspruch 2, wobei das TIMP eine Anleitung und Warnungen für den Benutzer aufweist, wobei die Anleitung und Warnungen auf der Grundlage der Zustandsübergangskriterien zur Darstellung bestimmt werden.

10. Dynamisches Anreizpaket-Erzeugungssystem nach Anspruch 9, wobei die Anleitung und Warnungen Anweisungen aufweisen, um autonome Funktionen des Körpers des Benutzers unter Verwendung freiwilliger Handlungen, die zum Spielen des DIMP erforderlich sind, anzuregen.

11. Dynamisches Anreizpaket-Erzeugungssystem nach Anspruch 1, zudem aufweisend ein Regelungsmodul, wobei:
das Regelungsmodul so ausgebildet ist, dass es nach Erhalt einer Änderung an einem der validierten digitalen Therapeutika ein therapeutisches Profil des geänderten digitalen Therapeutikums auf der Grundlage eines vorbestimmten Satzes von Validierungsregeln aktualisiert, wobei:
wenn das geänderte digitale Therapeutikum mit dem vorbestimmten Satz von Validierungsregeln konform ist, das aktualisierte therapeutische Profil erzeugt wird, und
andernfalls die Änderung an einem der validierten digitalen Therapeutika abgelehnt wird, so dass das geänderte digitale Therapeutikum als für einen therapeutischen Zweck geeignet verifiziert wird.

12. Computerprogrammprodukt (CPP), das ein Programm mit Anweisungen aufweist, das auf einem nicht-transitorischen, computerlesbaren Medium konkret verkörpert ist, wobei die Anweisungen, wenn sie auf einem Prozessor ausgeführt werden, den Prozessor veranlassen, Operationen eines dynamischen Anreizpaket-Erzeugungssystems (100) für immersive Behandlungsmechanismen auszuführen, wobei das System ausgebildet ist zum:
Gewähren von Zugriff auf einen Datenspeicher (135), aufweisend:
eine Mehrzahl validierter digitaler Therapeutika (155),
eine Mehrzahl von therapeutischen Profilen (280), die jeweils einem der Mehrzahl von validierten digitalen Therapeutika zugeordnet sind,
ein Protokoll (255) mit Benutzereingaben, die dem Benutzer zugeordnet sind, wobei das Protokoll aufweist: eine Historie von Benutzerantworten auf Protokollierungsaufforderungen und eine Historie von Interaktionen des Benutzers mit Medien in einem Anreizzustand,
ein Benutzerprofil (140), das einem Benutzer zugeordnet ist und eine zeitlich verteilte Historie des Benutzers und Attribute des Benutzers aufweist, einschließlich Pathologieattributen und Motivationsattributen, und
eine Mehrzahl von Medienprofilen (145), die einer Mehrzahl von öffentlich zugänglichen Wild-Spielen zumindest für nicht-medizinische Zwecke (150) zugeordnet sind, wobei die Wild-Spiele eines oder mehrere der folgenden Elemente beinhalten: Spiele, Musik oder Videos, wobei die Mehrzahl von Medienprofilen bestimmten Medienobjekten von der Mehrzahl von Wild-Spielen entspricht und vorbestimmte Medienattribute und digitale Therapieübergangspunkte beinhaltet;
Betreiben einer mit dem Datenspeicher gekoppelten Bewertungs-Engine (125), wobei die Bewertungs-Engine ausgebildet ist, das dem Benutzer zugeordnete Benutzerprofil auf der Grundlage des aus dem Datenspeicher abgerufenen Protokolls mit Benutzereingaben zu erzeugen, wobei das Erzeugen des Benutzerprofils ein Erzeugen eines anfänglichen Benutzerprofils und ein Aktualisieren des Benutzerprofils zumindest auf der Grundlage der Benutzerantworten auf Protokollierungsaufforderungen aufweist; und
Betreiben einer mit dem Datenspeicher gekoppelten Engine zur Erzeugung medizinischer Medienpakete (130), wobei die Engine zur Erzeugung medizinischer Medienpakete ausgebildet ist, ein dynamisches Anreizmedienpaket (DIMP) zu erzeugen, aufweisend:
ein oder mehrere von der Mehrzahl validierter digitaler Therapeutika, die ausgewählt werden auf der Grundlage: des Benutzerprofils, einschließlich der zeitlich verteilten Historie, der Pathologieattribute und der Motivationsattribute; der Mehrzahl von therapeutischen Profilen; und eines Medienprofils aus der Mehrzahl von Medienprofilen, das einem von dem Benutzer ausgewählten Wild-Spiel aus der Mehrzahl von Wild-Spielen zugeordnet ist,
Zustandsübergangskriterien, die auf der Grundlage des Benutzerprofils und der validierten digitalen Therapeutika in dem DIMP erzeugt werden, wobei:
die Zustandsübergangskriterien mindestens eines von einem Zeitkriterium und einem Spielstoppkriterium beinhalten, um einen Übergang zwischen einem Anreizmodus und einem therapeutischen Modus zu ermöglichen, und
wenn die Zustandsübergangskriterien erfüllt sind, ein Betriebszustand des DIMP von dem Anreizmodus in den therapeutischen Modus übergeht,
Erleichtern eines Betriebs des DIMP auf einem Benutzergerät (106), das mit dem dynamischen Anreizpaket-Erzeugungssystem (100) entfernt gekoppelt ist, wobei in einer Laufzeit des DIMP:
in dem Anreizmodus das von dem Benutzer ausgewählte Wild-Spiel gespielt wird,
wenn Laufzeitbedingungen die Zustandsübergangskriterien erfüllt haben, das DIMP von dem Anreizmodus in einen therapeutischen Modus wechselt, und die Engine zur Erzeugung medizinischer Medienpakete ein therapeutisches immersives Medienpaket (TIMP) erzeugt, das einen empfohlenen Satz validierter Medieninhalte aufweist, die auf der Grundlage der therapeutischen Profile der validierten digitalen Therapeutika und auf der Grundlage des Benutzerprofils ausgewählt wurden, wobei der empfohlene Satz validierter Medieninhalte auf einer therapeutischen Schnittstelle auf dem Benutzergerät präsentiert wird,
in dem therapeutischen Modus die Engine zur Erzeugung medizinischer Medienpakete einen interventionellen Überwachungsinhalt (IMC), der das TIMP aufweist, erzeugt, sodass das DIMP von der Ausführung des von dem Benutzer ausgewählten Wild-Spiels zu den validierten digitalen Therapeutika umschaltet, und
nach Empfang einer Antwort des IMC die Bewertungs-Engine das Benutzerprofil auf der Grundlage der empfangenen Antwort aktualisiert, sodass ein nachfolgendes DIMP auf der Grundlage der empfangenen Antwort erzeugt wird.

13. Computerprogrammprodukt nach Anspruch 12, wobei der validierte Medieninhalt einen Satz von Fragen aufweist.

14. Computerprogrammprodukt nach Anspruch 12, wobei die Anweisungen den Prozessor zudem veranlassen, eine dynamische Anreizpaket-Zustandsmaschine (DIPSM) zu betreiben, die so ausgebildet ist, dass sie während einer Laufzeit des DIMP
(a) mindestens einen Übergangspunkt in dem DIMP identifiziert,
(b) die Laufzeit des DIMP von dem Anreizmodus in den therapeutischen Modus überführt,
(c) das TIMP auf dem Benutzergerät darstellt, und
(d) ein Signal an die Bewertungs-Engine sendet, um das Benutzerprofil auf der Grundlage eines Ergebnisses des TIMP zu aktualisieren.

15. Dynamisches Anreizpaket-Erzeugungssystem nach Anspruch 14, wobei die DIPSM zudem ausgebildet ist, einen Satz vorbestimmter Laufzeitparameter des DIMP auf der Grundlage des TIMP und des therapeutischen Profils des validierten Medieninhalts in dem TIMP und dem Benutzerprofil anzupassen.

## Revendications

1. Système de génération dynamique de paquets d'incitation (100) configuré pour générer de manière dynamique des mécanismes de traitement immersifs pouvant être utilisés pour l'auto-traitement, comprenant :
une mémoire de données (135) comprenant :
une pluralité de thérapies numériques validées (155),
une pluralité de profils thérapeutiques (280) associés chacun à l'une de la pluralité de thérapies numériques validées,
un journal (255) des entrées utilisateur associées à l'utilisateur, le journal comprenant : un historique de réponses de l'utilisateur à des invites du journal, et un historique des interactions de l'utilisateur avec des médias dans un état d'incitation,
un profil utilisateur (140) associé à un utilisateur et comprenant un historique distribué dans le temps de l'utilisateur et des attributs de l'utilisateur, y compris des attributs pathologiques et des attributs de motivation, et
une pluralité de profils médiatiques (145) associés à une pluralité de jeux vidéo sauvages accessibles au public au moins pour un usage non médical (150), les jeux vidéo sauvages comprenant un ou plusieurs des jeux, de la musique ou des vidéos, la pluralité de profils médiatiques correspondant à des objets médiatiques spécifiques de la pluralité de jeux vidéo sauvages et comprenant des attributs médiatiques prédéterminés et des points de transition de thérapie numérique ;
un moteur d'évaluation (125) couplé à la mémoire de données, dans lequel le moteur d'évaluation est configuré pour générer le profil utilisateur associé à l'utilisateur sur la base du journal des entrées utilisateur récupéré à partir de la mémoire de données, dans lequel la génération du profil utilisateur comprend une génération d'un profil utilisateur initial et une mise à jour du profil utilisateur au moins sur la base des réponses de l'utilisateur à des invites du journal ; et
un moteur de génération de paquets de médias médicaux (130) couplé à la mémoire de données, dans lequel le moteur de génération de paquets de médias médicaux est configuré pour générer un paquet de médias d'incitation dynamique (DIMP) comprenant :
une ou plusieurs parmi la pluralité de thérapies numériques validées sélectionnées sur la base : du profil utilisateur, y compris l'historique distribué dans le temps, les attributs pathologiques et les attributs de motivation ; de la pluralité de profils thérapeutiques ; et d'un profil médiatique, parmi la pluralité de profils médiatiques, associé à un jeu vidéo sauvage sélectionné par l'utilisateur parmi la pluralité de jeux vidéo sauvages,
des critères de transition d'état générés sur la base du profil utilisateur et des thérapies numériques validées dans le DIMP, dans lequel :
les critères de transition d'état comprennent au moins un critère temporel et un critère d'arrêt de jeu pour permettre une transition entre un mode d'incitation et un mode thérapeutique, et
lorsque les critères de transition d'état sont remplis, un état de fonctionnement du DIMP passe du mode d'incitation au mode thérapeutique,
dans lequel :
le DIMP est configuré pour être traité dans un dispositif utilisateur (106) couplé à distance au système de génération dynamique de paquets d'incitation, et dans lequel, dans un environnement d'exécution du DIMP,
en mode d'incitation, le jeu vidéo sauvage sélectionné par l'utilisateur est joué,
lorsque des conditions d'exécution ont satisfait aux critères de transition d'état, le DIMP passe du mode d'incitation à un mode thérapeutique, et le moteur de génération de paquets de médias médicaux génère un paquet de médias immersifs thérapeutiques (TIMP) comprenant un ensemble recommandé de contenus médiatiques validés sélectionnés sur la base des profils thérapeutiques des thérapies numériques validées et sur la base du profil utilisateur, dans lequel l'ensemble recommandé de contenus médiatiques validés est présenté sur une interface thérapeutique sur le dispositif utilisateur,
en mode thérapeutique, le moteur de génération de paquets de médias médicaux génère un contenu de surveillance interventionnelle (IMC) comprenant le TIMP de telle sorte que le DIMP passe de l'exécution du jeu vidéo sauvage sélectionné par l'utilisateur aux thérapies numériques validées, et,
à la réception d'une réponse de l'IMC, le moteur d'évaluation met à jour le profil utilisateur sur la base de la réponse reçue, de telle sorte qu'un DIMP ultérieur soit généré sur la base de la réponse reçue.

2. Système de génération dynamique de paquets d'incitation selon la revendication 1, dans lequel le contenu médiatique validé comprend un ensemble de questions.

3. Système de génération dynamique de paquets d'incitation selon la revendication 1, comprenant en outre une machine à états de paquets d'incitation dynamique (DIPSM) configurée pour, pendant l'exécution du DIMP,
(a) identifier au moins un point de transition dans le DIMP,
(b) faire passer l'exécution du DIMP du mode d'incitation au mode thérapeutique,
(c) présenter le TIMP sur le dispositif utilisateur, et
(d) transmettre un signal au moteur d'évaluation afin de mettre à jour le profil utilisateur sur la base d'un résultat du TIMP.

4. Système de génération dynamique de paquets d'incitation selon la revendication 3, dans lequel la DIPSM est en outre configurée pour ajuster un ensemble de paramètres d'exécution prédéterminés du DIMP sur la base du TIMP et du profil thérapeutique du contenu médiatique validé dans le TIMP et du profil utilisateur.

5. Système de génération dynamique de paquets d'incitation selon la revendication 1, dans lequel le passage de l'exécution du jeu vidéo sauvage sélectionné par l'utilisateur aux thérapies numériques validées comprend une insertion d'une instruction dans le jeu vidéo sauvage.

6. Système de génération dynamique de paquets d'incitation selon la revendication 1, dans lequel le passage de l'exécution du jeu vidéo sauvage sélectionné par l'utilisateur aux thérapies numériques validées comprend une interruption de l'exécution du DIMP pendant la lecture du jeu vidéo sauvage.

7. Système de génération dynamique de paquets d'incitation selon la revendication 1, comprenant en outre une couche d'observation, dans lequel la couche d'observation est configurée pour
observer le comportement de l'utilisateur afin de collecter des informations métriques sur la santé, et,
solliciter des réponses de l'utilisateur sur la base des informations collectées.

8. Système de génération dynamique de paquets d'incitation selon la revendication 1, dans lequel :
le moteur de génération de paquets de médias médicaux est configuré pour, lorsque la présentation du DIMP n'est pas appropriée en raison de contraintes environnementales comprenant un environnement non adapté au jeu, générer de manière dynamique un DIMP simplifié comprenant le TIMP sur la base du profil utilisateur et d'une historique des DIMP joués.

9. Système de génération dynamique de paquets d'incitation selon la revendication 2, dans lequel le TIMP comprend des conseils et des avertissements à l'intention de l'utilisateur, dans lequel les conseils et les avertissements sont déterminés pour être présentés en fonction des critères de transition d'état.

10. Système de génération dynamique de paquets d'incitation selon la revendication 9, dans lequel les conseils et les avertissements comprennent des instructions visant à induire une fonction autonome du corps de l'utilisateur à l'aide d'une action volontaire requise pour jouer au DIMP.

11. Système de génération dynamique de paquets d'incitation selon la revendication 1, comprenant en outre un module de régulation, dans lequel :
le module de régulation est configuré pour, à la réception d'une modification apportée à l'une des thérapies numériques validées, mettre à jour un profil thérapeutique de la thérapie numérique modifiée sur la base d'un ensemble prédéterminé de règles de validation, dans lequel :
si la thérapie numérique modifiée est conforme à l'ensemble prédéterminé de règles de validation, le profil thérapeutique mis à jour est généré, et
sinon, la modification apportée à l'une des thérapies numériques validées est rejetée, de sorte que la thérapie numérique modifiée est vérifiée pour s'assurer qu'elle correspond à une intention thérapeutique.

12. Produit logiciel (CPP) comprenant un programme d'instructions concrètement incorporé sur un support lisible par ordinateur non transitoire dans lequel, lorsque les instructions sont exécutées sur un processeur, elles amènent le processeur à effectuer des opérations d'un système de génération dynamique de paquets d'incitation (100) pour des mécanismes de traitement immersifs, le système étant configuré pour :
fournir l'accès à une mémoire de données (135) comprenant :
une pluralité de thérapies numériques validées (155),
une pluralité de profils thérapeutiques (280) associés chacun à l'une de la pluralité de thérapies numériques validées,
un journal (255) des entrées utilisateur associés à l'utilisateur, le journal comprenant : un historique de réponses de l'utilisateur à des invites du journal, et un historique des interactions de l'utilisateur avec des médias dans un état d'incitation,
un profil utilisateur (140) associé à un utilisateur et comprenant un historique distribué dans le temps de l'utilisateur et des attributs de l'utilisateur, y compris des attributs pathologiques et des attributs de motivation, et
une pluralité de profils médiatiques (145) associés à une pluralité de jeux sauvages accessibles au public au moins pour un usage non médical (150), les jeux sauvages comprenant un ou plusieurs des jeux, de la musique ou des vidéos, la pluralité de profils médiatiques correspondant à des objets médiatiques spécifiques de la pluralité de jeux sauvages et comprenant des attributs médiatiques prédéterminés et des points de transition de thérapie numérique ;
faire fonctionner un moteur d'évaluation (125) couplé à la mémoire de données, dans lequel le moteur d'évaluation est configuré pour générer le profil utilisateur associé à l'utilisateur sur la base du journal des entrées utilisateur récupéré à partir de la mémoire de données, dans lequel la génération du profil utilisateur comprend une génération d'un profil utilisateur initial, et une mise à jour du profil utilisateur au moins sur la base des réponses de l'utilisateur à des invites du journal ; et
faire fonctionner un moteur de génération de paquets de médias médicaux (130) couplé à la mémoire de données, dans lequel le moteur de génération de paquets de médias médicaux est configuré pour générer un paquet de médias d'incitation dynamique (DIMP) comprenant :
une ou plusieurs parmi la pluralité de thérapies numériques validées sélectionnées sur la base : du profil utilisateur, y compris l'historique distribué dans le temps, les attributs pathologiques et les attributs de motivation ; de la pluralité de profils thérapeutiques ; et d'un profil médiatique, parmi la pluralité de profils médiatiques, associé à un jeu sauvage sélectionné par l'utilisateur parmi la pluralité de jeux sauvages,
des critères de transition d'état générés sur la base du profil utilisateur et des thérapies numériques validées dans le DIMP, dans lesquels :
les critères de transition d'état comprennent au moins un critère temporel et un critère d'arrêt de jeu pour permettre une transition entre un mode d'incitation et un mode thérapeutique, et
lorsque les critères de transition d'état sont remplis, un état de fonctionnement du DIMP passe du mode d'incitation au mode thérapeutique,
faciliter des opérations du DIMP sur un dispositif utilisateur (106) couplé à distance au système de génération dynamique de paquets d'incitation (100), dans lequel, dans un environnement d'exécution du DIMP :
en mode d'incitation, le jeu sauvage sélectionné par l'utilisateur est joué,
lorsque des conditions d'exécution ont satisfait aux critères de transition d'état, le DIMP passe du mode d'incitation au mode thérapeutique, et le moteur de génération de paquets de médias médicaux génère un paquet de médias immersifs thérapeutiques (TIMP) comprenant un ensemble recommandé de contenus médiatiques validés sélectionnés sur la base des profils thérapeutiques des thérapies numériques validées et sur la base du profil utilisateur, dans lequel l'ensemble recommandé de contenus médiatiques validés est présenté sur une interface thérapeutique sur le dispositif utilisateur,
en mode thérapeutique, le moteur de génération de paquets de médias médicaux génère un contenu de surveillance interventionnelle (IMC) comprenant le TIMP de telle sorte que le DIMP passe de l'exécution du jeu sauvage sélectionné par l'utilisateur aux thérapies numériques validées, et,
à la réception d'une réponse de l'IMC, le moteur d'évaluation met à jour le profil utilisateur sur la base de la réponse reçue, de telle sorte qu'un DIMP ultérieur soit généré sur la base de la réponse reçue.

13. Produit logiciel selon la revendication 12, dans lequel le contenu médiatique validé comprend un ensemble de questions.

14. Produit logiciel selon la revendication 12, dans lequel les instructions amènent en outre le processeur à faire fonctionner une machine à états de paquet d'incitation dynamique (DIPSM) configurée pour, pendant une exécution du DIMP,
(a) identifier au moins un point de transition dans le DIMP,
(b) faire passer l'exécution du DIMP du mode d'incitation au mode thérapeutique,
(c) présenter le TIMP sur le dispositif utilisateur, et
(d) transmettre un signal au moteur d'évaluation afin de mettre à jour le profil utilisateur sur la base d'un résultat du TIMP.

15. Système de génération dynamique de paquets d'incitation selon la revendication 14, dans lequel la DIPSM est en outre configurée pour ajuster un ensemble de paramètres d'exécution prédéterminés du DIMP sur la base du TIMP et du profil thérapeutique du contenu médiatique validé dans le TIMP et du profil utilisateur.
